# EUROPEAN PATENT APPLICATION

(11) **EP 4 744 660 A1**
(43) Date of publication of application: **20.05.2026**
(21) Application number: 24838915.7
(22) Date of filing: 08.08.2024
(51) Int. Cl.: A61K 31/437, C07D 519/00, C07D 471/04, A61K 9/08, A61K 9/10, A61K 9/14, A61P 3/04, A61P 3/10

(54) **GLP-1R AGONIST PREPARATION AND PREPARATION METHOD THEREFOR**

(30) Priority: 12.07.2023 CN 202310856937; 08.08.2023 US 202363531417 P; 01.05.2024 CN 202410544932; 22.05.2024 CN 202410644340
(71) Applicant: ASCLETIS PHARMA (CHINA) CO., LIMITED, 999077 Hong Kong (HK)
(72) Inventor: WU, Jingzi Jason, Hong Kong (CN); GUO, Ligang, Hong Kong (CN); DONG, Kunhua, Hong Kong (CN)
(74) Representative: J A Kemp LLP
(86) International application number: PCT/CN2024/110840
(87) International publication number: WO 2025/011664

(57) **Abstract**

Disclosed are long-acting injectable formulation and process for preparing the same. The formulation comprises a compound of Formula (I), a compound of Formula (II) or compound 6, stereoisomers, pharmaceutically acceptable salts or deuterated compounds thereof and at least one pharmaceutically acceptable carrier.

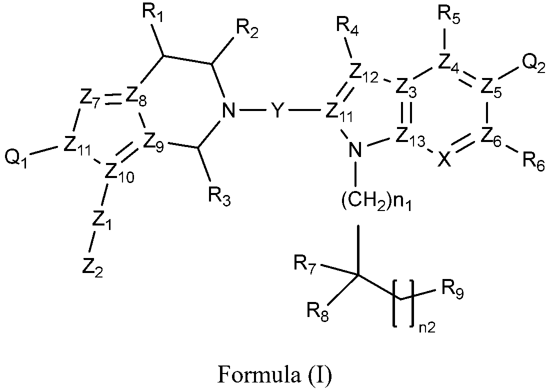

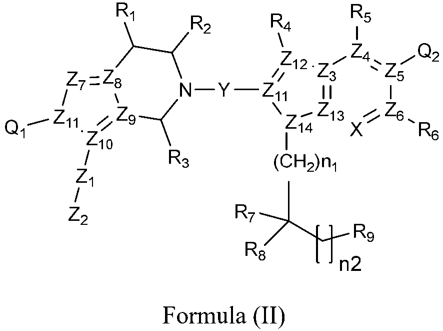

## Description

### CROSS-REFERENCE OF RELATED APPLICATIONS

The present disclosure claims all the benefits of the Chinese patent application No. 202410644340.1, entitled "Formulation Comprising GLP-1R Agonist and Process for Preparing The Same", filed on May 22, 2024 before the China National Intellectual Property Administration; the Chinese patent application No. 202410544932.6, entitled "Formulation Comprising GLP-1R Agonist and Process for Preparing The Same", filed on May 1, 2024 before the China National Intellectual Property Administration; the Chinese patent application No. 202310856937.8, entitled "Formulation Comprising GLP-1R Agonist and Process for Preparing The Same", filed on July 12, 2023 before the China National Intellectual Property Administration; and the U.S. provisional application No. 63/531,417, entitled "Formulation Comprising GLP-1R Agonist and Use Thereof", filed on August 8, 2023 before the U.S. Patent and Trademark Office, of which all the contents are incorporated herein by reference.

### FIELD

The present disclosure relates to the field of medicine, and more particularly, to a formulation comprising GLP-1R agonist and use thereof.

### BACKGROUND

Glucagon-like peptide-1 (GLP-1) is a peptide hormone secreted by enteroendocrine cells in the intestine in response to meals.GLP-1 is thought to play a role in postprandial blood glucose regulation by directly increasing meal-induced insulin secretion from pancreatic β-cells, and by delaying the transport of food through the intestine to promote satiety.GLP-1 mediates intracellular signaling via the GLP-1 receptor (GLP -1R) mediates intracellular signaling through the GLP-1 receptor, which belongs to a family of G-protein-coupled receptors present on cell membranes and can lead to accumulation of the second messenger cyclic adenosine monophosphate (cAMP) upon activation. Non-alcoholic steatohepatitis (NASH) can be associated with features of the metabolic syndrome, including obesity, type 2 diabetes, insulin resistance, and cardiovascular disease.

GLP-1R agonists are currently being extensively investigated for use in diseases associated with diabetes, obesity, and NASH. GLP-1R agonists include peptides such as exenatide, liraglutide, and dulaglutide, which are approved for the treatment of type 2 diabetes. Such peptides are administered primarily by subcutaneous injection. Oral GLP-1 agonists are also being studied for the treatment of type 2 diabetes. Some GLP-1R agonists, such as liraglutide, dulaglutide, and exenatide, are resistant to rapid degradation by dipeptidyl peptidase 4, resulting in longer half-lives than endogenous GLP-1.

There is a persistent need for compounds possessing desirable therapeutic profiles, metabolic profiles, and/or convenience of administration for the treatment of GLP-1R mediated diseases and disorders.

The present disclosure is directed to a long-acting GLP-1R agonist composition and the delivery of a therapeutic agent to transition the dosing schedule from a daily regimen to a weekly or monthly regimen, or even lower dosing frequencies, thereby enhancing patient privacy and satisfaction and improving compliance with therapeutic regimens.

Relative to oral formulations, long-acting injections exhibit stable plasma concentrations, prolonged duration of effective action for up to several weeks or even months, and reduced frequency of administration. The purposeful development of such formulations provides significant convenience for treatment while yielding substantial social benefits.

### SUMMARY

The long-acting injectable formulation provided by the present application exhibits stable plasma concentrations, a prolonged effective duration of drug action lasting for several weeks or even months and a reduced frequency of administration.

The present disclosure relates to a long-acting injectable formulation, comprising an active compound and at least a pharmaceutically acceptable carrier, wherein the active compound is a compound of Formula (I), stereoisomers, pharmaceutically acceptable salts or deuterated compounds thereof: 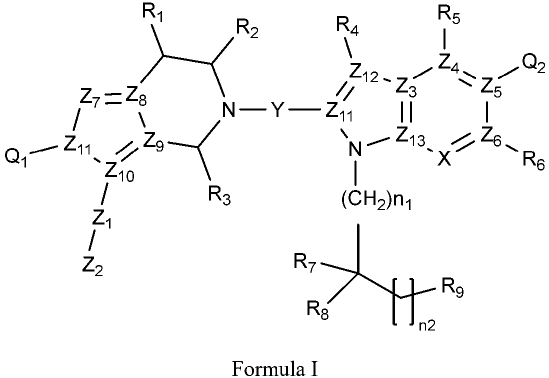 wherein
X is selected from the group consisting of -N= and -CR^{a}, wherein R^{a} is selected from the group consisting of hydrogen atom, halogen and C₁₋₆alkyl;
each of Z₃, Z₄, Z₅, Z₆, Z₇, Z₈, Z₉, Z₁₀, Z₁₁, Z₁₂ and Z₁₃ is independently selected from the group consisting of -N= and -CR^{a};
Q₁ is independently selected from the group consisting of C₆₋₁₀ aryl or 5-to 10-membered heteroaryl; wherein C₆₋₁₀aryl or 5- to 10-membered heteroaryl is optionally substituted by 1 to 5 substituents independently selected from the group consisting of halogen, C₁₋₆alkyl, C₁₋₆haloalkyl, and C₁₋₆alkoxy;
Q₂ is independently selected from the group consisting of 3- to 12-membered heterocyclic group and 5- to 10-membered heteroaryl group, wherein 3-to 12-membered heterocyclic group and 5- to 10-membered are optionally substituted by 1 to 3 substituents independently selected from the group consisting of halogen atoms, C₁₋₆alkyl, C₁₋₆haloalkyl, C₁₋₆alkoxy, and -NR^{Qa}R^{Qb}; and two C₁₋₆ alkyl groups together with carbon atoms to which they are attached form C₃₋₈carbon ring; and wherein R^{Qa} and R^{Qb} are each independently selected from the group consisting of hydrogen, C₁₋₆alkyl, C₁₋₆haloalkyl, and (C₁₋₆alkyl)carbonyl;
each of R₁, R₂ and R₃ is independently selected from the group consisting of hydrogen and C₁₋₆alkyl, wherein C₁₋₆alkyl is optionally substituted by one or more substituents each independently selected from the group consisting of halogen, C₁₋₆alkoxy and -OH;
each of R₄, R₅ and R₆ is independently selected from the group consisting of hydrogen, halogen and C₁₋₆alkyl;
each of R₇ and R₈ is independently selected from the group consisting of hydrogen and C₁₋₆alkyl, wherein C₁₋₆alkyl is optionally substituted by one or more substituents independently selected from the group consisting of halogen and C₃₋₁₅cycloalkyl, or wherein R₇ and R₈ together with carbon atoms to which they are attached form C₃₋₁₅carbocyclic ring; wherein C₃₋₁₅carbocyclic ring formed by R₇ and R₈ together is optionally substituted by 1 to 3 C₁₋₆alkyl, wherein C₁₋₆alkyl is optionally substituted by one or more substituents independently selected from the group consisting of halogen, hydroxyl, -NR^{7a}R^{7b}, C₁₋₆alkoxy, and 3- to 12-membered heterocyclic group, and wherein each of R^{7a} and R^{7b} is independently selected from the group consisting of hydrogen, C₁₋₆alkyl, and (C₁₋₆ alkyl)carbonyl;
n₁ is 0, 1, 2, or 3;
n₂ is 0, 1, 2, 3, 4 or 5;
R₉ is selected from the group consisting of 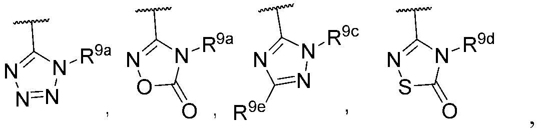 -CO₂R^{9f} and -C(=O)-NR^{9g}R^{9h}; and each of R^{9a}, R^{9b}, R^{9c}, R^{9d}, and R^{9g} is independently selected from the group consisting of hydrogen, C₁₋₆alkyl, and (C₁₋₆alkyl)carbonyl; wherein C₁₋₆alkyl group is optionally substituted by one or more substituents independently selected from the group consisting of halogen and C₁₋₆alkoxy, R^{9e} is hydrogen or C₁₋₆alkyl optionally substituted by one or more halogen atoms; wherein R^{9f} is independently selected from the group consisting of hydrogen and C₁₋₆alkyl; wherein R^{9h} is independently selected from the group consisting of hydrogen, C₁₋₆alkyl, (C₁₋₆alkyl)carbonyl, cyano, and -S(=O)ₙ₃-R⁹ⁱ; wherein n₃ is 0, 1 or 2; and R⁹ⁱ is C₁₋₆alkyl;
Z₁ is selected from the group consisting of 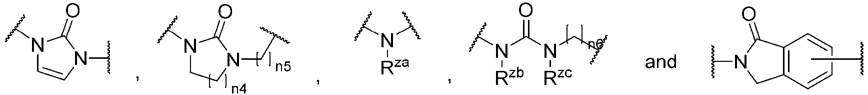
wherein R^{za} is selected from the group consisting of hydrogen, C₁₋₆alkyl and (C₁₋₆alkyl)carbonyl, each of R^{zb} and R^{zc} is independently selected from the group consisting of hydrogen and C₁₋₆alkyl; n₄ is 1, 2 or 3; and n₅ and n₆ are independently integers from 0 to 10;
Z₂ is selected from the group consisting of C₁₋₆alkyl, C₃₋₁₅cycloalkyl, 3-to 12-membered heterocyclyl, C₆₋₁₀aryl, and 5- to 10-membered heteroaryl, wherein C₃₋₁₅cycloalkyl, 3- to 12-membered heterocyclyl, C₆₋₁₀aryl, and 5- to 10-membered heteroaryl are independently and optionally substituted by one to five G; wherein G is selected from the group consisting of:
   a. oxo;
   b. halogen;
   c. cyano;
   d. -NR^{zd}R^{ze}; wherein R^{zd} and R^{ze} are independently selected from the group consisting of hydrogen, C₁₋₆alkyl, and (C₁₋₆alkyl)carbonyl; wherein C₁₋₆ alkyl is optionally substituted by one or more substituents independently selected from the group consisting of hydroxyl, halogen, and C₁₋₆alkoxy;
   e. -C(=O)-NR^{zf}R^{zg}; wherein each of R^{zf} and R^{zg} is independently selected from the group consisting of hydrogen, C₁₋₆alkyl, and (C₁₋₆alkyl)carbonyl; wherein C₁₋₆alkyl is optionally substituted by one or more substituents independently selected from the group consisting of hydroxyl, halogen, and C₁₋₆alkoxy;
   f. -S(=O)ₙ₇-R^{zh}; wherein n₇ is 0, 1 or 2; R^{zh} is independently selected from the group consisting of hydrogen and C₁₋₆alkyl;
   g. C₁₋₆alkyl; wherein the C₁₋₆alkyl is optionally substituted by one or more substituents independently selected from the group consisting of halogen, hydroxyl, -NR^{zi}R^{zj}, C₁₋₆alkoxy, and 3- to 12-membered heterocyclyl; wherein each of R^{zi} and R^{zj} is independently selected from the group consisting of hydrogen or C₁₋₆alkyl, and wherein 3- to 12-membered heterocyclyl is optionally substituted by one or more cyano, C₁₋₆alkyl and 3- to 12-membered heterocyclyl;
   h. C₁₋₆alkoxy; wherein C₁₋₆alkoxy is optionally substituted by one or more hydroxyl, halogen and C₁₋₆alkoxy;
   i. 3- to 12-membered heterocyclic; wherein 3- to 12-membered heterocyclic is optionally substituted by one or more substituents independently selected from the group consisting of C₁₋₆alkyl and (C₁₋₆alkyl)carbonyl;
   j. C₆₋₁₀aryl; wherein C₆₋₁₀aryl is optionally substituted by one or more (C₁₋₆ alkyl)carbonyl ; and
   k. 5- to 10-membered heteroaryl; wherein 5- to 10-membered heteroaryl is optionally substituted by one or more substituents independently selected from C₁₋₆alkyl, C₁₋₆alkoxy, -NR^{zk}R^{zl}, and 3- to 12-membered heterocyclic; wherein R^{zk} and R^{zl} are independently selected from the group consisting of hydrogen, C₁₋₆alkyl, and (C₁₋₆alkyl)carbonyl, and wherein 3- to 12-membered heterocyclic is optionally substituted by one or more substituents independently selected from the group consisting of C₁₋₆alkyl and C₁₋₆alkoxy.

The present disclosure relates to a long-acting injectable formulation, comprising an active compound and at least a pharmaceutically acceptable carrier, wherein the active compound is a compound of Formula (II), stereoisomers, pharmaceutically acceptable salts or deuterated compounds thereof: 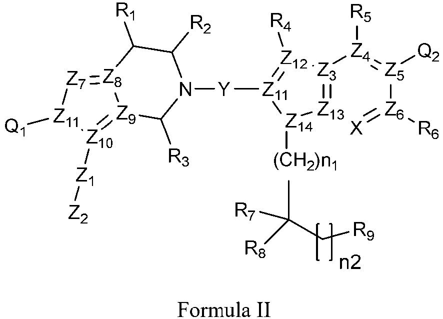 wherein
X is selected from the group consisting of -N= and -CR^{a}, wherein R^{a} is selected from the group consisting of hydrogen atom, halogen and C₁₋₆alkyl;
each of Z₃, Z₄, Z₅, Z₆, Z₇, Z₈, Z₉, Z₁₀, Z₁₁, Z₁₂ and Z₁₃ is independently selected from the group consisting of -N= and -CR^{a};
Q₁ is independently selected from the group consisting of C₆₋₁₀ aryl or 5-to 10-membered heteroaryl; wherein C₆₋₁₀aryl or 5- to 10-membered heteroaryl is optionally substituted by 1 to 5 substituents independently selected from the group consisting of halogen, C₁₋₆alkyl, C₁₋₆haloalkyl, and C₁₋₆alkoxy;
Q₂ is independently selected from the group consisting of 3- to 12-membered heterocyclic group and 5- to 10-membered heteroaryl group, wherein 3-to 12-membered heterocyclic group and 5- to 10-membered are optionally substituted by 1 to 3 substituents independently selected from the group consisting of halogen atoms, C₁₋₆alkyl, C₁₋₆haloalkyl, C₁₋₆alkoxy, and -NR^{Qa}R^{Qb}; and two C₁₋₆ alkyl groups together with carbon atoms to which they are attached form C₃₋₈carbon ring; and wherein R^{Qa} and R^{Qb} are each independently selected from the group consisting of hydrogen, C₁₋₆alkyl, C₁₋₆haloalkyl, and (C₁₋₆alkyl)carbonyl;
each of R₁, R₂ and R₃ is independently selected from the group consisting of hydrogen and C₁₋₆alkyl, wherein C₁₋₆alkyl is optionally substituted by one or more substituents each independently selected from the group consisting of halogen, C₁₋₆alkoxy and -OH;
each of R₄, R₅ and R₆ is independently selected from the group consisting of hydrogen, halogen and C₁₋₆alkyl;
each of R₇ and R₈ is independently selected from the group consisting of hydrogen and C₁₋₆alkyl, wherein C₁₋₆alkyl is optionally substituted by one or more substituents independently selected from the group consisting of halogen and C₃₋₁₅cycloalkyl, or wherein R₇ and R₈ together with carbon atoms to which they are attached form C₃₋₁₅carbocyclic ring; wherein C₃₋₁₅carbocyclic ring formed by R₇ and R₈ together is optionally substituted by 1 to 3 C₁₋₆alkyl, wherein C₁₋₆alkyl is optionally substituted by one or more substituents independently selected from the group consisting of halogen, hydroxyl, -NR^{7a}R^{7b}, C₁₋₆alkoxy, and 3- to 12-membered heterocyclic group, and wherein each of R^{7a} and R^{7b} is independently selected from the group consisting of hydrogen, C₁₋₆alkyl, and (C₁₋₆ alkyl)carbonyl;
n₁ is 0, 1, 2, or 3;
n₂ is 0, 1, 2, 3, 4 or 5;
R₉ is selected from the group consisting of 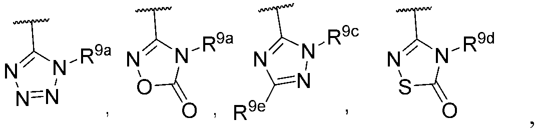 -CO₂R^{9f} and -C(=O)-NR^{9g}R^{9h}; and each of R^{9a}, R^{9b}, R^{9c}, R^{9d}, and R^{9g} is independently selected from the group consisting of hydrogen, C₁₋₆alkyl, and (C₁₋₆alkyl)carbonyl; wherein C₁₋₆alkyl group is optionally substituted by one or more substituents independently selected from the group consisting of halogen and C₁₋₆alkoxy, R^{9e} is hydrogen or C₁₋₆alkyl optionally substituted by one or more halogen atoms; wherein R^{9f} is independently selected from the group consisting of hydrogen and C₁₋₆alkyl; wherein R^{9h} is independently selected from the group consisting of hydrogen, C₁₋₆alkyl, (C₁₋₆alkyl)carbonyl, cyano, and -S(=O)ₙ₃-R⁹ⁱ; wherein n₃ is 0, 1 or 2; and R⁹ⁱ is C₁₋₆alkyl;
Z₁ is selected from the group consisting of 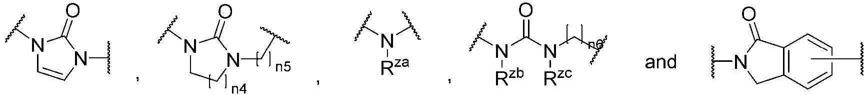
wherein R^{za} is selected from the group consisting of hydrogen, C₁₋₆alkyl and (C₁₋₆alkyl)carbonyl, each of R^{zb} and R^{zc} is independently selected from the group consisting of hydrogen and C₁₋₆alkyl; n₄ is 1, 2 or 3; and n₅ and n₆ are independently integers from 0 to 10;
Z₂ is selected from the group consisting of C₁₋₆alkyl, C₃₋₁₅cycloalkyl, 3-to 12-membered heterocyclyl, C₆₋₁₀aryl, and 5- to 10-membered heteroaryl, wherein C₃₋₁₅cycloalkyl, 3- to 12-membered heterocyclyl, C₆₋₁₀aryl, and 5- to 10-membered heteroaryl are independently and optionally substituted by one to five G; wherein G is selected from the group consisting of:
   a. oxo;
   b. halogen;
   c. cyano;
   d. -NR^{zd}R^{ze}; wherein R^{zd} and R^{ze} are independently selected from the group consisting of hydrogen, C₁₋₆alkyl, and (C₁₋₆alkyl)carbonyl; wherein C₁₋₆ alkyl is optionally substituted by one or more substituents independently selected from the group consisting of hydroxyl, halogen, and C₁₋₆alkoxy;
   e. -C(=O)-NR^{zf}R^{zg}; wherein each of R^{zf} and R^{zg} is independently selected from the group consisting of hydrogen, C₁₋₆alkyl, and (C₁₋₆alkyl)carbonyl; wherein C₁₋₆alkyl is optionally substituted by one or more substituents independently selected from the group consisting of hydroxyl, halogen, and C₁₋₆alkoxy;
   f. -S(=O)ₙ₇-R^{zh}; wherein n₇ is 0, 1 or 2; R^{zh} is independently selected from the group consisting of hydrogen and C₁₋₆alkyl;
   g. C₁₋₆alkyl; wherein the C₁₋₆alkyl is optionally substituted by one or more substituents independently selected from the group consisting of halogen, hydroxyl, -NR^{zi}R^{zj}, C₁₋₆alkoxy, and 3- to 12-membered heterocyclyl; wherein each of R^{zi} and R^{zj} is independently selected from the group consisting of hydrogen or C₁₋₆alkyl, and wherein 3- to 12-membered heterocyclyl is optionally substituted by one or more cyano, C₁₋₆alkyl and 3- to 12-membered heterocyclyl;
   h. C₁₋₆alkoxy; wherein C₁₋₆alkoxy is optionally substituted by one or more hydroxyl, halogen and C₁₋₆alkoxy;
   i. 3- to 12-membered heterocyclic; wherein 3- to 12-membered heterocyclic is optionally substituted by one or more substituents independently selected from the group consisting of C₁₋₆alkyl and (C₁₋₆alkyl)carbonyl;
   j. C₆₋₁₀aryl; wherein C₆₋₁₀aryl is optionally substituted by one or more (C₁₋₆ alkyl)carbonyl ;
   k. 5- to 10-membered heteroaryl; wherein 5- to 10-membered heteroaryl is optionally substituted by one or more substituents independently selected from C₁₋₆alkyl, C₁₋₆alkoxy, -NR^{zk}R^{zl}, and 3- to 12-membered heterocyclic; wherein R^{zk} and R^{zl} are independently selected from the group consisting of hydrogen, C₁₋₆alkyl, and (C₁₋₆alkyl)carbonyl, and wherein 3- to 12-membered heterocyclic is optionally substituted by one or more substituents independently selected from the group consisting of C₁₋₆alkyl and C₁₋₆alkoxy; and
   l. -P(O)R_{g1}R_{g2}; wherein R_{g1} and R_{g2} are each independently selected from the group consisting of hydrogen, C₁₋₆alkyl, and C₃₋₈cycloalkyl.

The present disclosure relates to a long-acting injectable formulation, comprising an active compound and at least a pharmaceutically acceptable carrier, wherein the active compound is compound 6, stereoisomers, pharmaceutically acceptable salts or deuterated compounds thereof: 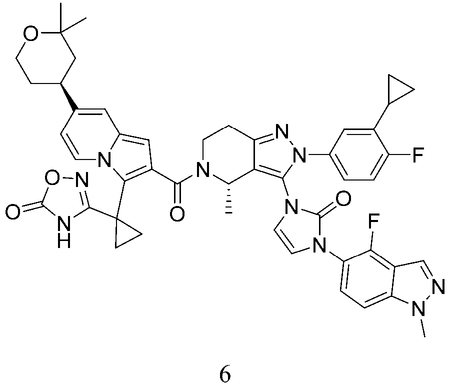 wherein preferably the pharmaceutically acceptable salts are selected from the group consisting of sodium salts, potassium salts, calcium salts, and magnesium salts.

In another aspect, the present disclosure relates to a method for treating GLP-1-mediated diseases or conditions, comprising administering to a subject an effective amount of the formulation of the present disclosure.

### I. DETAILED DESCRIPTION

In the following description, certain specific details are included to provide a thorough understanding of the various disclosed embodiments. However, those skilled in the relevant art will recognize that embodiments may be practiced without one or more of these specific details, but with other methods, components, materials, and the like.

Unless otherwise claimed in this disclosure, throughout the specification and the claims that follow, the words "comprise" and "comprising" are to be construed in an open, inclusive sense, i.e., "including, but not limited to."

As used in the present disclosure and the appended claims, the singular reference without an indication of quantity includes the plural reference unless the context clearly dictates otherwise.

Reference throughout this specification to "one embodiment" or "an embodiment" or "in another embodiment" or "in some embodiments" is meant to include in at least one embodiment specific reference elements, structures, or features relevant to those described in that embodiment. Thus, the appearances of the phrases "in one embodiment" or "in an embodiment" or "in another embodiment" or "in some embodiments" in various places throughout the specification are not necessarily all referring to the same embodiment. Furthermore, the specific elements, structures, or features may be combined in any suitable manner in one or more embodiments.

It is to be understood that the singular form used in the specification of the present disclosure and the appended claims include plural referents unless the context clearly dictates otherwise. Thus, for example, reference to a sustained release tablet comprising "a pharmaceutically acceptable excipient" includes one pharmaceutically acceptable excipient, or two or more pharmaceutically acceptable excipients.

### II. Definition

Unless defined otherwise, all technical and scientific terms used herein have the same meaning as commonly understood by one of ordinary skill in the art. The dashes at the front or end of the chemical group are for convenience to indicate the point of attachment to the parent molecule; Chemical groups may be described with or without one or more dashes without losing their ordinary meaning. A prefix such as "Cᵤ₋ᵥ" or "Cᵤ-Cᵥ" indicates that the following groups have from u to v carbon atoms, where u and v are integers. For example, "C₁₋₆alkyl" or "C₁-C₆alkyl" means that the alkyl group has 1 to 6 carbon atoms.

"Alkyl" is a monovalent or divalent linear or branched saturated hydrocarbon group. For example, the alkyl group may have 1 to 10 carbon atoms (i.e., C₁₋₁₀alkyl) or 1 to 8 carbon atoms (i.e., C₁₋₈alkyl) or 1 to 6 carbon atoms (i.e., C₁₋₆alkyl) or 1 to 4 carbon atoms (i.e., C₁₋₄alkyl). Examples of alkyl groups include, but are not limited to, methyl (Me, -CH₃), ethyl (Et, -CH₂CH₃), 1-propyl (n-Pr, n-propyl, CH₂CH₂CH₃), 2-propyl (i-Pr, i-Propyl, -CH(CH₃)₂), 1-butyl (n-Bu, n-butyl, -CH₂CH₂CH₂CH₃), 2-methyl-1-propyl (i-Bu, i-butyl, -CH₂CH(CH₃)₂), 2-butyl (s-Bu, s-butyl, -CH(CH₃)CH₂CH₃), 2-methyl-2-propyl (t-Bu, t-butyl, -C(CH₃)₃), 1-pentyl (n-pentyl, -CH₂CH₂CH₂CH₃), 2-pentyl (-CH(CH₃)CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), 1-hexyl (-CH₂CH₂CH₂CH₃), 2-hexyl (-CH(CH₃)CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃), and octyl (-(CH₂)₇CH₃). Alkyl groups may be unsubstituted or substituted.

"Alkenyl" is a monovalent or divalent linear or branched hydrocarbon group having at least one carbon-carbon double bond. For example, an alkenyl group may have 2 to 8 carbon atoms (i.e., C₂₋₈alkenyl) or 2 to 6 carbon atoms (i.e., C₂₋₆alkenyl) or 2 to 4 carbon atoms (i.e., C₂₋₄alkenyl). Examples of alkenyl groups include, but are not limited to, -CH=CH₂, -CH₂CH=CH₂, and -CH₂-CH=CH-CH₃. Alkenyl groups may be unsubstituted or substituted.

"Alkynyl" is a monovalent or divalent linear or branched hydrocarbon group having at least one carbon-carbon triple bond. For example, an alkynyl group may have 2 to 8 carbon atoms (i.e., C₂₋₈alkynyl) or 2 to 6 carbon atoms (i.e., C₂₋₆alkynyl) or 2 to 4 carbon atoms (i.e., C₂₋₄alkynyl). Examples of alkynyl include, but are not limited to, -C≡CH, -CH₂C≡CH, and -CH₂-C≡C-CH₃. Alkynyl groups may be unsubstituted or substituted.

"Alkoxyalkyl" is an alkoxy group attached to an alkyl group as defined above such that the alkyl group is bivalent. For example, C₂₋₆alkoxyalkyl includes -CH₂-OMe, -CH₂-O-iPr, -CH₂-CH₂-OMe, -CH₂-CH₂-O-CH₂-CH₃, and -CH₂-CH₂-O-tBu. Alkoxyalkyl groups may be unsubstituted or substituted.

"Halogen" refers to fluorine (-F), chlorine (-Cl), bromine (-Br) and iodine (-I).

"Haloalkyl" is an alkyl group as defined herein, wherein one or more hydrogen atoms of the alkyl group are independently substituted with one halogen, which halogen may be the same or different, such that the alkyl group is divalent. Alkyl and halogen may be any of those described above. In some embodiments, haloalkyl determines the number of carbon atoms of the alkyl moiety, for example, C₁₋₄haloalkyl includes CF₃, CH₂F, CHF₂, CH₂CF₃, CH₂CH₂CF₃, CCl₂CH₂CH₃, and C(CH₃)₂(CF₂H). Haloalkyl groups may be unsubstituted or substituted.

"Aryl" refers to a monovalent or divalent mono- or polycondensed all-carbon aromatic ring system in which the ring is aromatic. For example, in some embodiments, one aryl group has 6 to 20 carbon atoms, 6 to 14 carbon atoms, 6 to 12 carbon atoms, or 6 to 10 carbon atoms. Aryl groups include one phenyl group. Aryl also includes a plurality of condensed ring systems (e.g., ring systems consisting of 2, 3, or 4 rings) having about 9 to 20 carbon atoms, wherein the plurality of rings are aromatic. Where valence requirements permit, the rings of multiple condensed ring systems may be interconnected by fused bonds. It will also be appreciated that when referring to a member aryl group (such as an aryl group of 6 to 10 members) of an atomic range, the atomic range refers to the total ring atoms of the aryl group. For example, six member aryl groups include phenyl and ten member aryl groups include naphthyl. Non-limiting examples of aryl groups include, but are not limited to, phenyl, naphthyl, anthracenyl, and the like. Aryl groups may be unsubstituted or substituted.

"5- to 10-membered aromatic heterocycle" or "heteroaromatic ring" refers to a single aromatic ring having an atom other than at least one carbon in the ring, wherein the atom is selected from the group consisting of oxygen, nitrogen, and sulfur; "Heteroaryl" also includes a plurality of condensed ring systems having at least one such aromatic ring, the plurality of condensed ring systems being further described below. Thus, "heteroaryl" includes monoaromatic rings of about 1 to 6 carbon atoms and about 1 to 4 heteroatoms selected from the group consisting of oxygen, nitrogen and sulfur. Sulfur and nitrogen atoms may also be present in oxidized form as long as the ring is aromatic. Exemplary heteroaryl ring systems include, but are not limited to, pyridyl, pyrimidinyl, oxazolyl, or furyl. "Heteroaryl" also includes a plurality of condensed ring systems (e.g., ring systems consisting of 2, 3, or 4 rings) in which a heteroaryl as defined above is condensed with one or more rings selected from the group consisting of heteroaryl (to form, e.g., 1, 8-naphthyridinyl) and aryl (to form, e.g., benzimidazolyl or indazolyl) to form a plurality of condensed ring systems. Thus, a heteroaryl group (a single aromatic ring or multiple condensed ring systems) can have about 1 to 20 carbon atoms and about 1 to 6 heteroatoms within the heteroaryl ring. For example, tetrazolyl has 1 carbon atom and 4 nitrogen heteroatoms in the ring. Where valence requirements permit, the rings of multiple condensed ring systems may be interconnected by fused bonds. It will be appreciated that each ring of a plurality of condensed ring systems may be interconnected in any order. It should be understood that the point of attachment of the heteroaryl or heteroaryl multiple condensation ring system can be any suitable atom of the heteroaryl or heteroaryl multiple condensation ring system, including carbon atoms and heteroatoms (e.g., nitrogen). It is also to be understood that when reference is made to a member heteroaryl group (e.g., a 5 to 10 member heteroaryl group) of an atomic range, the atomic range is for the total ring atoms of the heteroaryl group, including carbon atoms and heteroatoms. It is also to be understood that rings of multiple condensed ring systems may include aryl rings fused to a heterocyclic ring having a saturated or partially unsaturated bond (e.g., a 3-, 4-, 5-, 6-, or 7-membered ring) having about 1 to 6 cyclic carbon atoms and about 1 to 3 cyclic heteroatoms selected from oxygen, nitrogen, and sulfur in the ring. For example, 5-membered heteroaryl includes thiazolyl and 10-membered heteroaryl includes quinolinyl. Exemplary heteroaryl groups include, but are not limited to, pyridyl, pyrrolyl, pyrazinyl, pyrimidinyl, pyrazolyl, thienyl, indolyl, imidazolyl, oxazolyl, isoxazolyl, thiazolyl, furanyl, oxadiazolyl, thiazolyl, quinolinyl, isoquinolinyl, benzothiazolyl, benzoxazolyl, indazolyl, quinoxazolyl, quinazolyl, benzofuranyl, benzimidazolyl, thiophenyl, pyrrolo [2, 3-b]pyridyl, quinazolinyl-4(3H)-one, triazolyl, and tetrazolyl. Heteroaryl groups may be unsubstituted or substituted.

"Cycloalkyl" is a monovalent or divalent single all-carbocyclic ring or multiple condensed all-carbocyclic ring systems, wherein the ring in each example is a non-aromatic saturated or unsaturated ring. For example, in some embodiments, a cycloalkyl group has 3 to 12 carbon atoms, 3 to 10 carbon atoms, 3 to 8 carbon atoms, 3 to 6 carbon atoms, 3 to 5 carbon atoms, or 3 to 4 carbon atoms. Exemplary monocyclic cycloalkyl groups include cyclopropyl, cyclobutyl, cyclopentyl, cyclohexyl, cycloalkenyl, cycloheptyl, cycloheptenyl, and cyclooctyl. Cycloalkyl also includes multiple condensed ring systems having about 7 to 12 carbon atoms (e.g., ring systems including 2 rings). Where valence requirements permit, the rings of multiple condensed ring systems may be interconnected by a fusion bond, a spiral bond, or a bridging bond. Exemplary polycyclic cycloalkyl groups include octahydropentene, bicyclic[2.2.1]heptane, bicyclo[2.2.2]octane, bicyclo[2.2]oct-2-ene and spiro[2.5]octane. The cycloalkyl group may be unsubstituted or substituted.

As used herein, "heterocycle" or "heterocycle" or "heterocyclyl group" refers to a single saturated or partially unsaturated non-aromatic ring or non-aromatic polycyclic ring system having at least one heteroatom in the ring (i.e., at least one cyclic (i.e., cyclic) heteroatom selected from oxygen, nitrogen, and sulfur). Unless otherwise specified, heterocyclic groups have from 3 to about 20 cyclic atoms, e.g., from 3 to 12 cyclic atoms, e.g., from 4 to 12 cyclic atoms, from 4 to 10 cyclic atoms, or from 3 to 8 cyclic atoms, or from 3 to 6 cyclic atoms, or from 4 to 6 cyclic atoms, or from 4 to 5 cyclic atoms. Thus, the term includes single saturated or partially unsaturated rings (e.g., 3, 4, 5, 6, or 7-membered rings) having about 1 to 6 cyclic carbon atoms and about 1 to 3 cyclic heteroatoms, the heteroatoms in these rings being selected from the group consisting of oxygen, nitrogen, and sulfur. Where valence requirements permit, the rings of multiple condensed rings, such as bicyclic heterocycles, may be interconnected by fused bonds, spiral bonds, and bridged bonds. Heterocycles include, but are not limited to, azacycle, aziridine, imidazolidine, morpholine, oxirane, oxirane, thiacycle, piperazine, piperidine. Pyrazolidine, piperidine, pyrrolidine, pyrrolidone, tetrahydrofuran, tetrahydrothiophene, dihydropyridine, tetrahydropyridine, quinuclidine, 2-oxo-6-azaspiro[3. 3]heptan-6-yl, 6-oxa-1-azaspiro[3.3]heptan-1-yl, 2-thia-6-azaspiro[3.3]heptan-6-yl, 2,6-diazaspiro[3.3]heptan-2-yl, 2-azabicyclo[3.1.0]hex-2-yl, 3-azabicyclo[3.0]hexyl, 2-azabicyclo[2.1.1]hexyl, 2-azabicyclo[2.2.1]hept-2-yl, 4-azaspiro[2.4]heptyl, 5-azaspiro[2.4]heptyl, and the like. Heterocyclyl groups may be unsubstituted or substituted.

As used herein, "substituted" means wherein one or more hydrogen atoms of a group are independently substituted with one or more substituents (e.g., 1, 2, 3, or 4 or more), as shown.

"Compounds of the present disclosure" include compounds disclosed herein, e.g., compounds of the present disclosure include compounds of Formula (I), including compounds of the Examples. In some embodiments, "compounds of the present disclosure" include compounds of Formula (I).

"Pharmaceutically acceptable excipients" include, but are not limited to, any adjuvants, carriers, excipients, lubricants, sweeteners, diluents, preservatives, dyes/colorants, flavoring agents, surfactants, wetting agents, dispersing agents, suspending agents, stabilizers, isotonic agents, solvents or emulsifiers, which have been approved by the U.S. Food and Drug Administration for use in humans or livestock.

As used herein, a "therapeutically effective amount" or "effective amount" refers to an amount that is effective to elicit a desired biological or medical response, including an amount of a compound that, when administered to a subject for treating a disease, is sufficient to affect the treatment of the disease. The effective amount will vary depending on the compound, the disease and its severity, as well as the age, weight, and other factors of the subject to be treated. An effective amount may include a range of amounts. As understood in the art, an effective amount may be one or more doses, i.e., one or more doses may be required to achieve the desired therapeutic end point. An effective amount can be considered in the case of administration of one or more therapeutic agents, and a single agent can be considered to be administered in an effective amount if a desired or beneficial result is or has been achieved with one or more other agents. Due to the combined effects of the compounds (e.g., additive or synergistic effects), the appropriate dosage of any co-administered compound may be selectively reduced.

As used herein, "co-administration" means administration of a unit dose of a compound of the present disclosure before or after administration of a unit dose of one or more additional therapeutic agents, e.g., administration of a compound of the present disclosure within a few seconds, minutes, or hours of administration of one or more additional therapeutic agents. For example, in some embodiments, a unit dose of a compound of the present disclosure is administered first, followed by a unit dose of one or more additional therapeutic agents within a few seconds or minutes. Alternatively, in other embodiments, a unit dose of one or more additional therapeutic agents is administered first, followed by a unit dose of a compound of the present disclosure within seconds or minutes. In some embodiments, a unit dose of a compound of the present disclosure is administered first, followed by a unit dose of one or more additional therapeutic agents after a few hours (e.g., 1-12 hours). In other embodiments, a unit dose of one or more additional therapeutic agents is administered first, followed by a unit dose of a compound of the present disclosure after a few hours (e.g., 1-12 hours). Co-administration of a compound disclosed herein with one or more additional therapeutic agents generally refers to simultaneous or sequential administration of a compound disclosed herein and one or more additional therapeutic agents such that a therapeutically effective amount of each agent is present in the subject's body.

Also provided are pharmaceutically acceptable salts, hydrates, solubles, isomeric forms, polymorphs, and prodrugs of the compounds described herein.

"Pharmaceutically acceptable" or "physiologically acceptable" refers to compounds, salts, compositions, dosage forms and other materials useful in the preparation of pharmaceutical compositions suitable for veterinary or human pharmaceutical use.

The compounds described herein may be prepared and/or formulated as pharmaceutically acceptable salts or, where appropriate, as free bases. A pharmaceutically acceptable salt is a non-toxic salt of a free base form of a compound that has the desired pharmacological activity of the free base. These salts may be extracted from inorganic or organic acids or bases. For example, a compound containing a basic nitrogen can be prepared as a pharmaceutically acceptable salt by contacting the compound with an inorganic or organic acid. Non-limiting examples of pharmaceutically acceptable salts include sulfates, pyrosulfates, disulfates, sulfites, disulfates, phosphates, monohydrogenphosphates, dihydrogenphosphates, metaphosphates, pyrophosphates. Chloride, bromide, iodide, acetate, propionate, decanoate, octanoate, acrylate, formate, isobutyrate, hexanoate, heptanoate, propoxide, oxalate, malonate, ferrite, sebacate, fumarate. Maleate, butyne-1, 4-diacid salt, hexyne-1, 6-diacid salt, benzoate, chlorobenzoate, toluate, dinitrobenzoate, hydroxybenzoate, methoxybenzoate, phthalate, sulfonate, methanesulfonate, propanesulfonate. Benzene sulfonate, xylene sulfonate, naphthalene-1-sulfonate, naphthalene-2-sulfonate, phenylacetate, phenylpropionate, phenylbutyrate, citrate, lactate, gamma-hydroxybutyrate, glycolate, tartrate and mandelate. A list of other suitable pharmaceutically acceptable salts can be found in Remington: The Science and Practice of Pharmacy, 21st Edition, Lippincott Wiliams and Wilkins, Philadelphia, Pa., 2006.

Examples of "pharmaceutically acceptable salts" of the compounds disclosed herein also include salts from suitable bases such as alkali metals (e.g., sodium, potassium), alkaline earth metals (e.g., magnesium), ammonium, and N (C₁-C₄alkyl)⁴⁺. Also included are base addition salts, such as sodium or potassium salts.

Also provided are compounds described herein, or pharmaceutically acceptable salts, isomers, or mixtures thereof, wherein 1 to n hydrogen atoms attached to a carbon atom may be substituted with a deuterium atom or D, wherein n is the number of hydrogen atoms in the molecule. As is known in the art, a deuterium atom is a non-radioactive isotope of a hydrogen atom. Such compounds may increase the resistance to metabolism and may therefore be used to increase the half-life of a compound described herein or a pharmaceutically acceptable salt, isomer, or mixture thereof when administered to mammal. See "Foster, "Deuterium Isotope Effects in Studies of Drug Metabolism", Trends Pharmacol. Sci., 5(12):524-527 (1984)"; Such compounds are synthesized by methods well known in the art, for example using starting materials in which one or more hydrogen atoms are substituted with deuterium.

Examples of isotopes that may be incorporated into the disclosed compounds also include isotopes of hydrogen, carbon, nitrogen, oxygen, phosphorus, fluorine, chlorine, and iodine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F , ³⁶Cl, ¹²³I and ¹²⁵I. Substitution with positron emission isotopes such as ¹¹C, ¹⁸F, ¹⁵O and 1³N can be used in positron emission topography (PET) studies to examine substrate receptor occupancy. Isotopically labeled compounds of formula (I-A-1) can generally be prepared by conventional techniques known to those skilled in the art, or by procedures analogous to those described in the Examples below, using the appropriate isotopically labeled reagent in place of the previously used non-labeled reagent.

The compounds of the embodiments disclosed herein, or pharmaceutically acceptable salts thereof, may contain one or more asymmetric centers and thus may produce enantiomeric, diastereomeric, and other stereoisomeric forms, which may be defined in absolute stereochemistry as (R)- or (S)-, or, in the case of amino acids, as (D)- or (L)-. The present disclosure is intended to include all such possible isomers, as well as their racemates and optically pure forms. Optically active (+) and (-), (R) and (S), or (D) and (L)- isomers may be prepared with chiral syntheses or chiral reagent, or resolved by conventional techniques, such as chromatography and fractional crystallization. Conventional techniques for preparing/separating individual enantiomers include chiral synthesis from suitable optically pure precursors, or resolution of racemates (or racemates of salts or derivatives) using, for example, chiral high pressure liquid chromatography (HPLC). When the compounds described herein contain olefinic double bonds or other centers of geometric asymmetry, such compounds are intended to include E and Z geometric isomers unless otherwise specified. Likewise, all isomeric forms are included. When a compound is represented in its chiral form, it is to be understood that this embodiment includes, but is not limited to, specific diastereomeric or enantiomerically enriched forms. If not specified but chirality is present, it is understood that this embodiment is for a particular diastereomeric or enantiomerically enriched form; Or a racemic or scalar mixture of such compounds. As used herein, "scalar mixture" refers to a mixture of stereoisomers in a ratio other than 1: 1.

As used herein, "stereoisomers" refer to compounds bound by the same atoms but having different three-dimensional structures, which are not interchangeable. The present disclosure contemplates various stereoisomers and mixtures thereof, and includes "enantiomers", which refer to two stereoisomers whose molecules are non-superimposable mirror images of one another.

As used herein, "tautomer" refers to a proton that is transferred from one atom of one molecule to another atom of the same molecule. In some embodiments, the present disclosure includes tautomers of the compounds.

As used herein, "solvate" refers to the result of the interaction of a solvent and a compound. Also provided are solvates of salts of the compounds described herein. Also provided are hydrates of the compounds described herein.

As used herein, "hydrate" refers to a compound of the invention that is chemically bound to one or more water molecules.

"Prophylaxis" or "prevention" refers to any treatment of a disease or condition that results in the non-progression of the clinical symptoms of the disease or condition. In some embodiments, a compound can be administered to a subject (including a human) at risk of a disease or disorder or a family history.

As used herein, "prodrug" refers to a derivative of a drug that is converted to the parent drug according to some chemical or enzymatic route after administration to the human body. In some embodiments, a prodrug is a biologically active derivative of a drug that is converted to a biologically active parent drug according to certain chemical or enzymatic routes after administration to a human.

As used herein, "treatment" or "treating" or "treat" refers to a method of obtaining a beneficial or desired result. For purposes of the present disclosure, beneficial or desirable results include, but are not limited to, alleviating symptoms and/or alleviating the extent of symptoms and/or preventing worsening of symptoms associated with a disease or condition. In one embodiment, "treatment" or "treating" includes one or more of : a) inhibiting the disease or condition (e.g., reducing one or more symptoms caused by the disease or condition, and/or reducing the degree of the disease or condition); b) slowing or arresting the development of one or more symptoms associated with the disease or condition (e.g., stabilizing the disease or condition, delaying the progression or progression of the disease or condition); and c) alleviating a disease or condition, e.g., causing regression of clinical symptoms, improving disease states, slowing disease progression, improving quality of life, and/or prolonging survival. As used herein, an individual "at risk" refers to an individual at risk of developing a disease in need of treatment. An individual "at risk" may or may not have a detectable disease or condition, and may or may not exhibit a detectable disease prior to the treatment methods described herein. "At risk" means that an individual has one or more so-called risk factors, which are measurable parameters associated with the development of a disease or condition, and which are known in the art to have a higher probability of developing a disease or condition in an individual having one or more of these risk factors than in an individual without these risk factors.

### III. Formulations

The present disclosure relates to a long-acting injectable formulation, comprising an active compound and at least a pharmaceutically acceptable carrier, wherein the active compound is a compound of Formula (I), stereoisomers, pharmaceutically acceptable salts or deuterated compounds thereof: 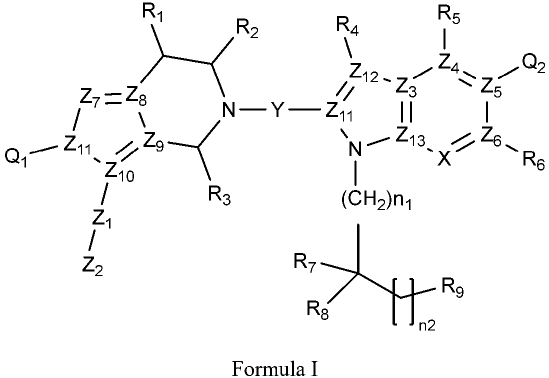 wherein
X is selected from the group consisting of -N= and -CR^{a}, wherein R^{a} is selected from the group consisting of hydrogen atom, halogen and C₁₋₆alkyl;
each of Z₃, Z₄, Z₅, Z₆, Z₇, Z₈, Z₉, Z₁₀, Z₁₁, Z₁₂ and Z₁₃ is independently selected from the group consisting of -N= and -CR^{a};
Q₁ is independently selected from the group consisting of C₆₋₁₀ aryl or 5-to 10-membered heteroaryl; wherein C₆₋₁₀aryl or 5- to 10-membered heteroaryl is optionally substituted by 1 to 5 substituents independently selected from the group consisting of halogen, C₁₋₆alkyl, C₁₋₆haloalkyl, and C₁₋₆alkoxy;
Q₂ is independently selected from the group consisting of 3- to 12-membered heterocyclic group and 5- to 10-membered heteroaryl group, wherein 3-to 12-membered heterocyclic group and 5- to 10-membered are optionally substituted by 1 to 3 substituents independently selected from the group consisting of halogen atoms, C₁₋₆alkyl, C₁₋₆haloalkyl, C₁₋₆alkoxy, and -NR^{Qa}R^{Qb}; and two C₁₋₆ alkyl groups together with carbon atoms to which they are attached form C₃₋₈carbon ring; and wherein R^{Qa} and R^{Qb} are each independently selected from the group consisting of hydrogen, C₁₋₆alkyl, C₁₋₆haloalkyl, and (C₁₋₆alkyl)carbonyl;
each of R₁, R₂ and R₃ is independently selected from the group consisting of hydrogen and C₁₋₆alkyl, wherein C₁₋₆alkyl is optionally substituted by one or more substituents each independently selected from the group consisting of halogen, C₁₋₆alkoxy and -OH;
each of R₄, R₅ and R₆ is independently selected from the group consisting of hydrogen, halogen and C₁₋₆alkyl;
each of R₇ and R₈ is independently selected from the group consisting of hydrogen and C₁₋₆alkyl, wherein C₁₋₆alkyl is optionally substituted by one or more substituents independently selected from the group consisting of halogen and C₃₋₁₅cycloalkyl, or wherein R₇ and R₈ together with carbon atoms to which they are attached form C₃₋₁₅carbocyclic ring; wherein C₃₋₁₅carbocyclic ring formed by R₇ and R₈ together is optionally substituted by 1 to 3 C₁₋₆alkyl, wherein C₁₋₆alkyl is optionally substituted by one or more substituents independently selected from the group consisting of halogen, hydroxyl, -NR^{7a}R^{7b}, C₁₋₆alkoxy, and 3- to 12-membered heterocyclic group, and wherein each of R^{7a} and R^{7b} is independently selected from the group consisting of hydrogen, C₁₋₆alkyl, and (C₁₋₆ alkyl)carbonyl;
n₁ is 0, 1, 2, or 3;
n₂ is 0, 1, 2, 3, 4 or 5;
R₉ is selected from the group consisting of 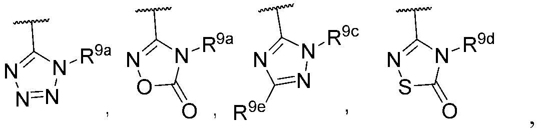 -CO₂R^{9f} and -C(=O)-NR^{9g}R^{9h}; and each of R^{9a}, R^{9b}, R^{9c}, R^{9d}, and R^{9g} is independently selected from the group consisting of hydrogen, C₁₋₆alkyl, and (C₁₋₆alkyl)carbonyl; wherein C₁₋₆alkyl group is optionally substituted by one or more substituents independently selected from the group consisting of halogen and C₁₋₆alkoxy, R^{9e} is hydrogen or C₁₋₆alkyl optionally substituted by one or more halogen atoms; wherein R^{9f} is independently selected from the group consisting of hydrogen and C₁₋₆alkyl; wherein R^{9h} is independently selected from the group consisting of hydrogen, C₁₋₆alkyl, (C₁₋₆alkyl)carbonyl, cyano, and -S(=O)ₙ₃-R⁹ⁱ; wherein n₃ is 0, 1 or 2; and R⁹ⁱ is C₁₋₆alkyl;
Z₁ is selected from the group consisting of 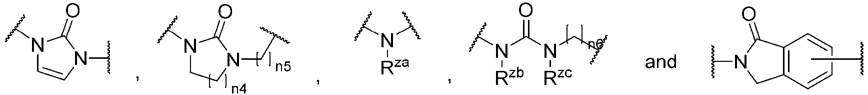
wherein R^{za} is selected from the group consisting of hydrogen, C₁₋₆alkyl and (C₁₋₆alkyl)carbonyl, each of R^{zb} and R^{zc} is independently selected from the group consisting of hydrogen and C₁₋₆alkyl; n₄ is 1, 2 or 3; and n₅ and n₆ are independently integers from 0 to 10;
Z₂ is selected from the group consisting of C₁₋₆alkyl, C₃₋₁₅cycloalkyl, 3-to 12-membered heterocyclyl, C₆₋₁₀aryl, and 5- to 10-membered heteroaryl, wherein C₃₋₁₅cycloalkyl, 3- to 12-membered heterocyclyl, C₆₋₁₀aryl, and 5- to 10-membered heteroaryl are independently and optionally substituted by one to five G; wherein G is selected from the group consisting of:
   a. oxo;
   b. halogen;
   c. cyano;
   d. -NR^{zd}R^{ze}; wherein R^{zd} and R^{ze} are independently selected from the group consisting of hydrogen, C₁₋₆alkyl, and (C₁₋₆alkyl)carbonyl; wherein C₁₋₆ alkyl is optionally substituted by one or more substituents independently selected from the group consisting of hydroxyl, halogen, and C₁₋₆alkoxy;
   e. -C(=O)-NR^{zf}R^{zg}; wherein each of R^{zf} and R^{zg} is independently selected from the group consisting of hydrogen, C₁₋₆alkyl, and (C₁₋₆alkyl)carbonyl; wherein C₁₋₆alkyl is optionally substituted by one or more substituents independently selected from the group consisting of hydroxyl, halogen, and C₁₋₆alkoxy;
   f. -S(=O)ₙ₇-R^{zh}; wherein n₇ is 0, 1 or 2; R^{zh} is independently selected from the group consisting of hydrogen and C₁₋₆alkyl;
   g. C₁₋₆alkyl; wherein the C₁₋₆alkyl is optionally substituted by one or more substituents independently selected from the group consisting of halogen, hydroxyl, -NR^{zi}R^{zj}, C₁₋₆alkoxy, and 3- to 12-membered heterocyclyl; wherein each of R^{zi} and R^{zj} is independently selected from the group consisting of hydrogen or C₁₋₆alkyl, and wherein 3- to 12-membered heterocyclyl is optionally substituted by one or more cyano, C₁₋₆alkyl and 3- to 12-membered heterocyclyl;
   h. C₁₋₆alkoxy; wherein C₁₋₆alkoxy is optionally substituted by one or more hydroxyl, halogen and C₁₋₆alkoxy;
   i. 3- to 12-membered heterocyclic; wherein 3- to 12-membered heterocyclic is optionally substituted by one or more substituents independently selected from the group consisting of C₁₋₆alkyl and (C₁₋₆alkyl)carbonyl;
   j. C₆₋₁₀aryl; wherein C₆₋₁₀aryl is optionally substituted by one or more (C₁₋₆ alkyl)carbonyl ; and
   k. 5- to 10-membered heteroaryl; wherein 5- to 10-membered heteroaryl is optionally substituted by one or more substituents independently selected from C₁₋₆alkyl, C₁₋₆alkoxy, -NR^{zk}R^{zl}, and 3- to 12-membered heterocyclic; wherein R^{zk} and R^{zl} are independently selected from the group consisting of hydrogen, C₁₋₆alkyl, and (C₁₋₆alkyl)carbonyl, and wherein 3- to 12-membered heterocyclic is optionally substituted by one or more substituents independently selected from the group consisting of C₁₋₆alkyl and C₁₋₆alkoxy.

In another aspect, the present disclosure relates to a long-acting injectable formulation, comprising an active compound and at least a pharmaceutically acceptable carrier, wherein the active compound is a compound of Formula (II), stereoisomers, pharmaceutically acceptable salts or deuterated compounds thereof: 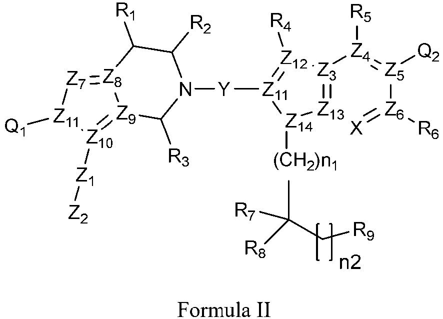 wherein
X is selected from the group consisting of -N= and -CR^{a}, wherein R^{a} is selected from the group consisting of hydrogen atom, halogen and C₁₋₆alkyl;
each of Z₃, Z₄, Z₅, Z₆, Z₇, Z₈, Z₉, Z₁₀, Z₁₁, Z₁₂ and Z₁₃ is independently selected from the group consisting of -N= and -CR^{a};
Q₁ is independently selected from the group consisting of C₆₋₁₀ aryl or 5-to 10-membered heteroaryl; wherein C₆₋₁₀aryl or 5- to 10-membered heteroaryl is optionally substituted by 1 to 5 substituents independently selected from the group consisting of halogen, C₁₋₆alkyl, C₁₋₆haloalkyl, and C₁₋₆alkoxy;
Q₂ is independently selected from the group consisting of 3- to 12-membered heterocyclic group and 5- to 10-membered heteroaryl group, wherein 3-to 12-membered heterocyclic group and 5- to 10-membered are optionally substituted by 1 to 3 substituents independently selected from the group consisting of halogen atoms, C₁₋₆alkyl, C₁₋₆haloalkyl, C₁₋₆alkoxy, and -NR^{Qa}R^{Qb}; and two C₁₋₆ alkyl groups together with carbon atoms to which they are attached form C₃₋₈carbon ring; and wherein R^{Qa} and R^{Qb} are each independently selected from the group consisting of hydrogen, C₁₋₆alkyl, C₁₋₆haloalkyl, and (C₁₋₆alkyl)carbonyl;
each of R₁, R₂ and R₃ is independently selected from the group consisting of hydrogen and C₁₋₆alkyl, wherein C₁₋₆alkyl is optionally substituted by one or more substituents each independently selected from the group consisting of halogen, C₁₋₆alkoxy and -OH;
each of R₄, R₅ and R₆ is independently selected from the group consisting of hydrogen, halogen and C₁₋₆alkyl;
each of R₇ and R₈ is independently selected from the group consisting of hydrogen and C₁₋₆alkyl, wherein C₁₋₆alkyl is optionally substituted by one or more substituents independently selected from the group consisting of halogen and C₃₋₁₅cycloalkyl, or wherein R₇ and R₈ together with carbon atoms to which they are attached form C₃₋₁₅carbocyclic ring; wherein C₃₋₁₅carbocyclic ring formed by R₇ and R₈ together is optionally substituted by 1 to 3 C₁₋₆alkyl, wherein C₁₋₆alkyl is optionally substituted by one or more substituents independently selected from the group consisting of halogen, hydroxyl, -NR^{7a}R^{7b}, C₁₋₆alkoxy, and 3- to 12-membered heterocyclic group, and wherein each of R^{7a} and R^{7b} is independently selected from the group consisting of hydrogen, C₁₋₆alkyl, and (C₁₋₆ alkyl)carbonyl;
n₁ is 0, 1, 2, or 3;
n₂ is 0, 1, 2, 3, 4 or 5;
R₉ is selected from the group consisting of 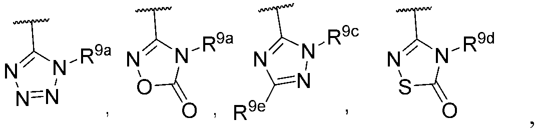 -CO₂R^{9f} and -C(=O)-NR^{9g}R^{9h}; and each of R^{9a}, R^{9b}, R^{9c}, R^{9d}, and R^{9g} is independently selected from the group consisting of hydrogen, C₁₋₆alkyl, and (C₁₋₆alkyl)carbonyl; wherein C₁₋₆alkyl group is optionally substituted by one or more substituents independently selected from the group consisting of halogen and C₁₋₆alkoxy, R^{9e} is hydrogen or C₁₋₆alkyl optionally substituted by one or more halogen atoms; wherein R^{9f} is independently selected from the group consisting of hydrogen and C₁₋₆alkyl; wherein R^{9h} is independently selected from the group consisting of hydrogen, C₁₋₆alkyl, (C₁₋₆alkyl)carbonyl, cyano, and -S(=O)ₙ₃-R⁹ⁱ; wherein n₃ is 0, 1 or 2; and R⁹ⁱ is C₁₋₆alkyl;
Z₁ is selected from the group consisting of 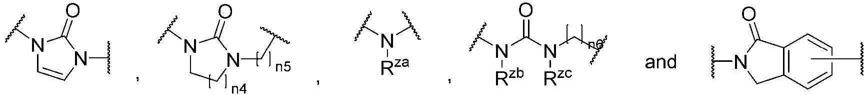
wherein R^{za} is selected from the group consisting of hydrogen, C₁₋₆alkyl and (C₁₋₆alkyl)carbonyl, each of R^{zb} and R^{zc} is independently selected from the group consisting of hydrogen and C₁₋₆alkyl; n₄ is 1, 2 or 3; and n₅ and n₆ are independently integers from 0 to 10;
Z₂ is selected from the group consisting of C₁₋₆alkyl, C₃₋₁₅cycloalkyl, 3-to 12-membered heterocyclyl, C₆₋₁₀aryl, and 5- to 10-membered heteroaryl, wherein C₃₋₁₅cycloalkyl, 3- to 12-membered heterocyclyl, C₆₋₁₀aryl, and 5- to 10-membered heteroaryl are independently and optionally substituted by one to five G; wherein G is selected from the group consisting of:
   a. oxo;
   b. halogen;
   c. cyano;
   d. -NR^{zd}R^{ze}; wherein R^{zd} and R^{ze} are independently selected from the group consisting of hydrogen, C₁₋₆alkyl, and (C₁₋₆alkyl)carbonyl; wherein C₁₋₆ alkyl is optionally substituted by one or more substituents independently selected from the group consisting of hydroxyl, halogen, and C₁₋₆alkoxy;
   e. -C(=O)-NR^{zf}R^{zg}; wherein each of R^{zf} and R^{zg} is independently selected from the group consisting of hydrogen, C₁₋₆alkyl, and (C₁₋₆alkyl)carbonyl; wherein C₁₋₆alkyl is optionally substituted by one or more substituents independently selected from the group consisting of hydroxyl, halogen, and C₁₋₆alkoxy;
   f. -S(=O)ₙ₇-R^{zh}; wherein n₇ is 0, 1 or 2; R^{zh} is independently selected from the group consisting of hydrogen and C₁₋₆alkyl;
   g. C₁₋₆alkyl; wherein the C₁₋₆alkyl is optionally substituted by one or more substituents independently selected from the group consisting of halogen, hydroxyl, -NR^{zi}R^{zj}, C₁₋₆alkoxy, and 3- to 12-membered heterocyclyl; wherein each of R^{zi} and R^{zj} is independently selected from the group consisting of hydrogen or C₁₋₆alkyl, and wherein 3- to 12-membered heterocyclyl is optionally substituted by one or more cyano, C₁₋₆alkyl and 3- to 12-membered heterocyclyl;
   h. C₁₋₆alkoxy; wherein C₁₋₆alkoxy is optionally substituted by one or more hydroxyl, halogen and C₁₋₆alkoxy;
   i. 3- to 12-membered heterocyclic; wherein 3- to 12-membered heterocyclic is optionally substituted by one or more substituents independently selected from the group consisting of C₁₋₆alkyl and (C₁₋₆alkyl)carbonyl;
   j. C₆₋₁₀aryl; wherein C₆₋₁₀aryl is optionally substituted by one or more (C₁₋₆ alkyl)carbonyl ;
   k. 5- to 10-membered heteroaryl; wherein 5- to 10-membered heteroaryl is optionally substituted by one or more substituents independently selected from C₁₋₆alkyl, C₁₋₆alkoxy, -NR^{zk}R^{zl}, and 3- to 12-membered heterocyclic; wherein R^{zk} and R^{zl} are independently selected from the group consisting of hydrogen, C₁₋₆alkyl, and (C₁₋₆alkyl)carbonyl, and wherein 3- to 12-membered heterocyclic is optionally substituted by one or more substituents independently selected from the group consisting of C₁₋₆alkyl and C₁₋₆alkoxy; and
   l. -P(O)R_{g1}R_{g2}; wherein R_{g1} and R_{g2} are each independently selected from the group consisting of hydrogen, C₁₋₆alkyl, and C₃₋₈cycloalkyl.

In some embodiments, the compound of Formula (I) has a structure of formula (III):

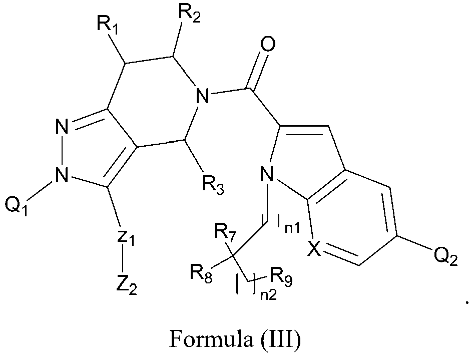

In some embodiments, he compound of Formula (III) has a structure of formula (V): 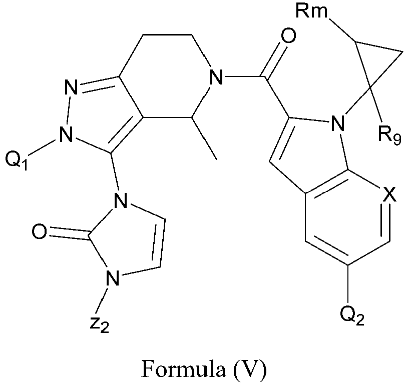 wherein Rm is C₁₋₆alkyl.

In some embodiments, the compound of Formula (II) has a structure of formula (IV):

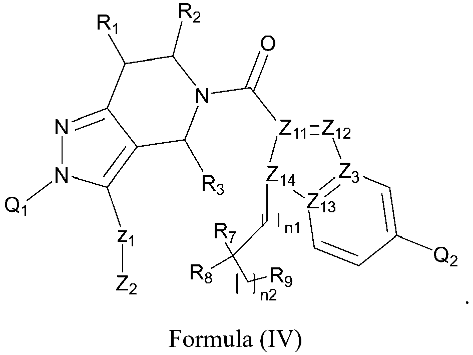

In some embodiments, the compound of Formula (IV) has a structure of formula (VI):

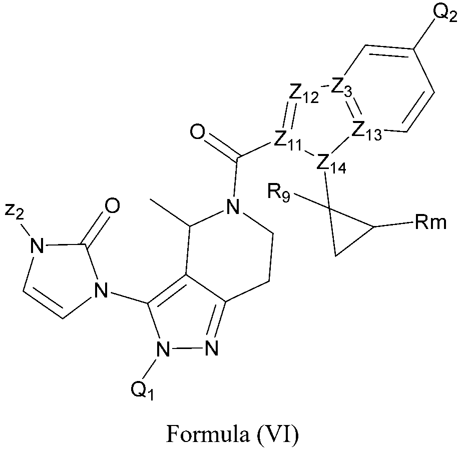

In some embodiments, the compound of Formula (II) is selected from the group consisting of: 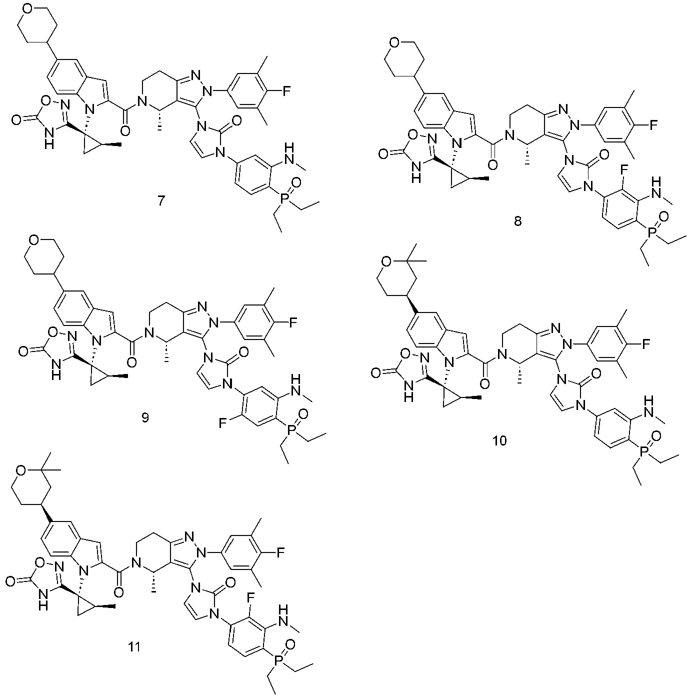 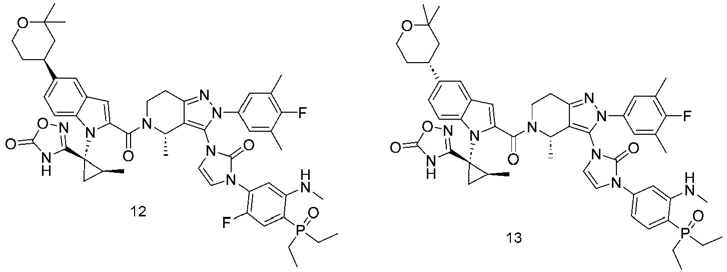 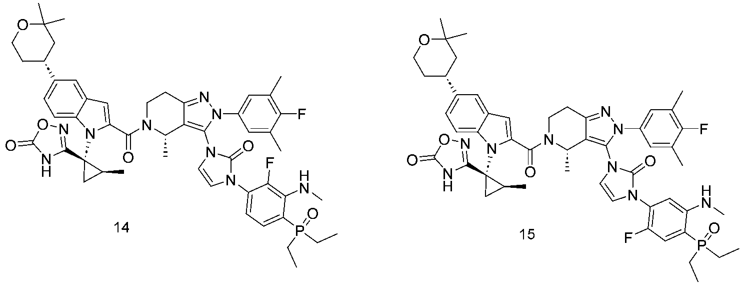 or stereoisomers, pharmaceutically acceptable salts or deuterated compounds thereof and at least one pharmaceutically acceptable carrier; preferably the pharmaceutically acceptable salts are selected from the group consisting of sodium salts, potassium salts, calcium salts, and magnesium salts.

In some embodiments, the active compound is compound 1: 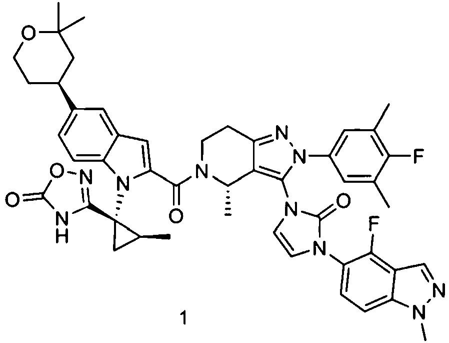 or stereoisomers, pharmaceutically acceptable salts or deuterated compounds thereof and at least one pharmaceutically acceptable carrier; preferably the pharmaceutically acceptable salts are selected from the group consisting of:
(1) sodium salts, comprising compound 2: 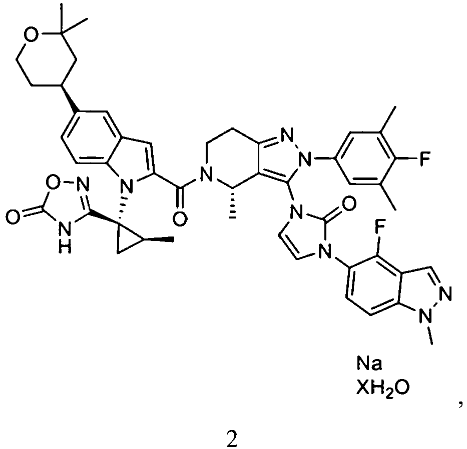 wherein X is number of crystal water and selected from the group consisting of 0, 0.5, 1, 2, and 3;
(2) potassium salts, comprising compound 3: 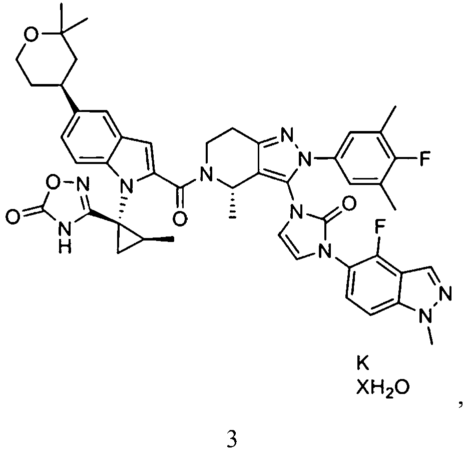 wherein X is number of crystal water and selected from the group consisting of 0, 0.5, 1, 2, and 3;
(3) calcium salts, comprising compound 4: 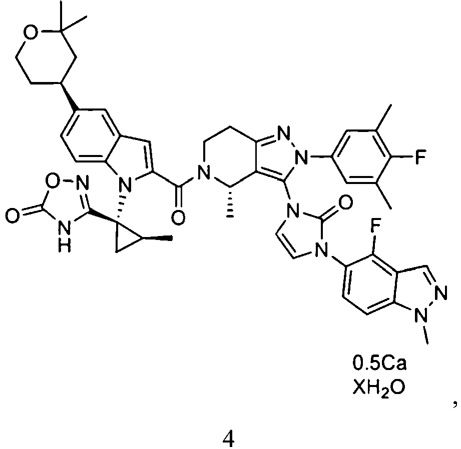 wherein X is number of crystal water and selected from the group consisting of 0, 0.5, 1, 2, and 3; and
(4) magnesium salts, comprising compound 5: 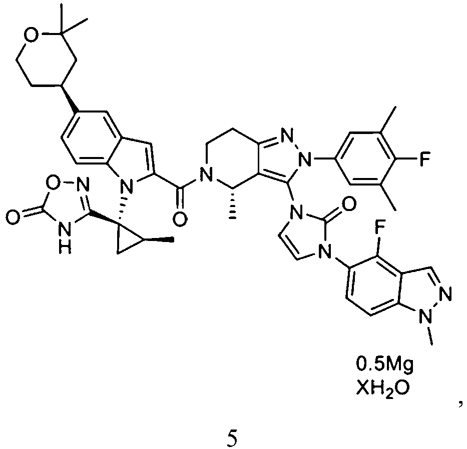 wherein X is number of crystal water and selected from the group consisting of 0, 0.5, 1, 2, and 3.

In another aspect, the present disclosure provides a long-acting injectable formulation, comprising an active compound and at least a pharmaceutically acceptable carrier, wherein the active compound is compound 6, stereoisomers, pharmaceutically acceptable salts or deuterated compounds thereof and at least a pharmaceutically acceptable carrier: 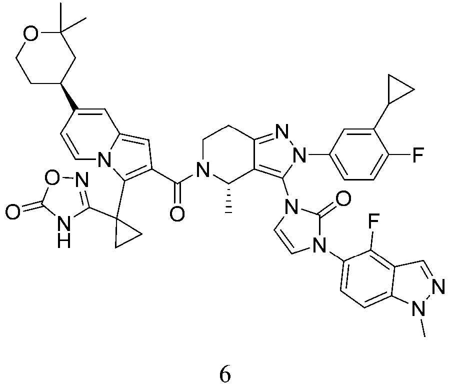 wherein preferably the pharmaceutically acceptable salts are selected from the group consisting of sodium salts, potassium salts, calcium salts, and magnesium salts.

In some embodiments, in accordance with the long-acting injectable formulation of the present application, the active compound is dissolved in a lipophilic carrier solution to form an oil solution, the lipophilic carrier solution comprises an oil solvent and an organic solvent;
preferably the active compound is selected from the group consisting of compounds 1, 6, 7-15, stereoisomers, pharmaceutically acceptable salts, and deuterated compounds thereof, more preferably is compound 1 or pharmaceutically acceptable salts thereof, and most preferably is compounds 1-5;
preferably the oil solvent is selected from the group consisting of castor oil, sesame oil, fatty acid glyceride, benzyl benzoate, benzyl alcohol, and any mixture thereof; more preferably the oil solvent is medium-chain fatty acid glyceride; and
preferably the organic solvent is benzyl alcohol or ethanol; more preferably the organic solvent is benzyl alcohol.
In some embodiments, in accordance with the oil solution of the present application, the concentration of the active compound or the pharmaceutically acceptable salts thereof in the formulation is 0.1 to 400 mg/mL, preferably 1 to 200 mg/mL;
wherein the weight ratio of the oil solvent to the organic solvent is from 6:4 to 9:1;
preferably the weight ratio of the oil solvent to the organic solvent isfrom 7:3 to 9:1; and
more preferably the weight ratio of the oil solvent to the organic solvent is 8:2.

In some embodiments, in accordance with the long-acting injectable formulation of the present application, the active compound is dissolved in a hydrophilic solvent to form a hydrophilic solution;
preferably the active compound is selected from the group consisting of compounds 1, 6, 7-15, stereoisomers, pharmaceutically acceptable salts, and deuterated compounds thereof, more preferably is compound 1 or pharmaceutically acceptable salts thereof, most preferably is compounds 1-5;
preferably the hydrophilic solvent comprises one of polyethylene glycol, organic solvent and water or any mixture thereof;
preferably the polyethylene glycol is selected from the group consisting of polyethylene glycol 300 and polyethylene glycol 400; more preferably the polyethylene glycol is polyethylene glycol 300; and
preferably the organic solvent is selected from the group consisting of benzyl alcohol, ethanol and N-methylpyrrolidone (NMP); more preferably the organic solvent is selected from the group consisting of benzyl alcohol and ethanol.

In some embodiments, in accordance with the hydrophilic solution of the present application, concentration of the active compound or pharmaceutically acceptable salts thereof in the formulation is 0.1 to 400 mg/mL, preferably 1 to 200 mg/mL;
wherein weight ratio of polyethylene glycol to the organic solvent is from 6:4 to 10:0;
more preferably weight ratio of polyethylene glycol to the organic solvent is from 7:3 to 10:0; even more preferably weight ratio of polyethylene glycol to the organic solvent is 9:1 to 10:0;
preferably weight ratio of polyethylene glycol 300 to the organic solvent is from 6:4 to 10:0; more preferably weight ratio of polyethylene glycol 300 to the organic solvent is from 7:3 to 10:0; even more preferably weight ratio of polyethylene glycol 300 to the organic solvent is 9:1 to 10:0; and
preferably weight ratio of polyethylene glycol 400 to the organic solvent is from 6:4 to 10:0; more preferably weight ratio of polyethylene glycol 400 to the organic solvent is from 7:3 to 10:0; even more preferably weight ratio of polyethylene glycol 400 to the organic solvent is 9:1 to 10:0.

In some embodiments, in accordance with the hydrophilic solution of the present application, the formulation further comprises a pH adjusting agent, wherein the pH adjusting agent is selected from the group consisting of sodium hydroxide, hydrochloric acid, citric acid, sodium citrate, phosphoric acid, sodium hydrogen phosphate, and any mixture thereof; preferably pH of the formulation is 4 to 10.

In some embodiments, in accordance with the hydrophilic solution of the present application, the formulation further comprises a release retardant, wherein the release retardant is selected from the group consisting of poly(lactic-co-glycolic acid) (PLGA) and polylactic acid (PLA), and weight ratio thereof in the hydrophilic solvent is 1% to 10%.

In some embodiments, in accordance with the long-acting injectable formulation, the active compound is dispersed in a stabilizer solution to form a suspension of drug-loaded microparticles or nanoparticles;
preferably the active compound is selected from the group consisting of compounds 1, 6, 7-15, stereoisomers, pharmaceutically acceptable salts, and deuterated compounds thereof, more preferably is compound 1 or pharmaceutically acceptable salts thereof, most preferably is compounds 1-5;
wherein the stabilizer solution is selected from the group consisting of one of polyethylene glycol, poloxamer, Tween, vitamin E polyethylene glycol succinate (TPGS), distearoylphosphatidylethanolamine (DSPE)-polyethylene glycol (PEG), polyvinylpyrrolidone, mannitol, hydroxypropyl methylcellulose, sodium carboxymethylcellulose, methylcellulose; and any combination thereof; preferably the stabilizer is selected from the group consisting of poloxamer and polyethylene glycol, wherein polyethylene glycol is polyethylene glycol 400 to 6000, more preferably polyethylene glycol 1000 to 4000, most preferably polyethylene glycol 3350, and poloxamer is poloxamer 407, poloxamer 338, or poloxamer 188.

In some embodiments, the suspension of drug-loaded microparticles or nanoparticles of the present application, the concentration of the active compound or pharmaceutically acceptable salts thereof in the formulation is 0.1 to 400 mg/mL, preferably 1 to 200 mg/mL;
wherein the weight ratio of poloxamer in the suspension of drug-loaded microparticles or nanoparticles is 1% to 10%,
wherein the weight ratio of polyethylene glycol in the suspension of drug-loaded microparticles or nanoparticles is 0.5% to 5%;
preferably the active ingredient in the microparticle or nanoparticle suspension exists in form of particles with a median particle size of about 0.1 to 10 µm; more preferably the median particle size range of the suspension of drug-loaded microparticles is 1 to 10 µm; even more preferably the median particle size range of the suspension of drug-loaded nanoparticles is 100 to 1000 nm.

In some embodiments, in accordance with the long-acting injectable formulation of the present application, the active compound is dispersed in a biodegradable polymer solution to prepare a polymer gel solution formulation, the polymer gel solution comprising a polymer gel material and an organic solvent;
preferably the active compound is selected from the group consisting of compounds 1, 6, 7-15, stereoisomers, pharmaceutically acceptable salts, and deuterated compounds thereof, more preferably is compound 1 or pharmaceutically acceptable salts thereof, most preferably is compounds 1-5;
wherein preferably the polymer gel material is selected from the group consisting of poly(lactic-co-glycolic acid) (PLGA), polylactic acid (PLA), polyoxyethylene-polycaprolactone block copolymer, and polyoxyethylene-polyoxypropylene block copolymer;
preferably the polymer gel material is poly(lactic-co-glycolic acid) (PLGA);
preferably the ratio of lactic acid to glycolic acid in the poly(lactic-co-glycolic acid) molecule is 75:25 or 50:50;
preferably the intrinsic viscosity of poly(lactic-co-glycolic acid) is 0.1 to 0.3 dL/g;
preferably the organic solvent is selected from the group consisting of N-methylpyrrolidone (NMP), dimethyl sulfoxide (DMSO), benzyl alcohol, benzyl benzoate, and any combination thereof;
preferably the concentration of the active compound or pharmaceutically acceptable salts thereof in the formulation is 0.1 to 400 mg/mL, preferably 1 to 200 mg/mL;
preferably the weight ratio of the poly(lactic-co-glycolic acid) in the polymer gel solution is 10% to 80%; and
preferably the weight ratio of the organic solvent in the polymer gel solution is 20% to 90%.

In accordance with the long-acting injectable formulation of the present application, the formulation is suitable for intramuscular or subcutaneous administration.

### IV. Method of Treatment

In accordance with another aspect of the present disclosure, the present disclosure relates to a pharmaceutical composition for treating GLP-1-mediated diseases or conditions, comprising T1 D, T2DM, prediabetes, idiopathic T1 D, LADA, EOD, YOAD, MODY, malnutrition-associated diabetes mellitus, gestational diabetes mellitus, hyperglycemia, insulin resistance, hepatic insulin resistance, impaired glucose tolerance, diabetic neuropathy, diabetic nephropathy, nephropathy, diabetic retinopathy, adipocyte dysfunction, visceral fat deposition, sleep apnea, obesity, eating disorders, weight gain due to use of other medications, excessive glucose consumption, dyslipidemia, hyperinsulinemia, NAFLD, NASH, fibrosis, cirrhosis, hepatocellular carcinoma, cardiovascular disease, atherosclerosis, coronary artery disease, peripheral vascular disease, hypertension, endothelial dysfunction, impaired vascular compliance, congestive heart failure, myocardial infarction, stroke, hemorrhagic stroke, ischemic stroke, traumatic brain injury, pulmonary hypertension, post angioplasty restenosis, intermittent claudication, postprandial lipemia, metabolic acidosis, ketoacidosis, arthritis, osteoporosis, Parkinson's disease, left ventricular hypertrophy, peripheral arterial disease, macular degeneration, cataract, glomerulosclerosis, chronic renal failure, metabolic syndrome, syndrome X, premenstrual syndrome, angina pectoris, thrombosis, atherosclerosis, transient ischemic attack, revascularization, impaired glucose metabolism, impaired fasting glucose, hyperuricemia, gout, erectile dysfunction, skin and connective tissue disorders, psoriasis, foot ulcers, ulcerative colitis, high apolipoprotein B lipoproteinemia, Alzheimer's disease, schizophrenia, cognitive impairment, inflammatory bowel disease, short bowel syndrome, Crohn's disease, colitis, irritable bowel syndrome, polycystic ovary syndrome and substance addiction.

The present disclosure is further illustrated below in connection with specific examples. It is to be understood that these examples are merely illustrative of the present disclosure and are not intended to limit the scope of the disclosure. Experimental methods not specified in the following examples generally follow conventional conditions or conditions suggested by the manufacturer.

Unless defined otherwise, all professional and scientific terms used herein have the same meaning as is familiar to those skilled in the art. In addition, any methods and materials similar or equivalent to those described herein can be applied to the disclosed methods. The preferred embodiments and materials described herein are exemplary only.

The above-mentioned features mentioned in the present disclosure, or the features mentioned in the embodiments, may be combined in any combination. All features disclosed in this patent specification may be used in any composition form, and each feature disclosed in the specification may be replaced by any alternative feature that may provide the same, equal or similar purpose. Thus, unless specifically stated otherwise, the disclosed features are merely general examples of equivalents or similar features.

### BRIEF DESCRIPTION OF DRAWINGS

FIG. 1 shows the PK curves of Compound 1 in Formulations 4, 8, and 12, respectively, after subcutaneous administration to SD rats.
FIG. 2 shows the PK curves of Compound 1 in Formulations 2-1, 5-2, and 11-1, respectively, after subcutaneous administration to SD rats.
FIG. 3 shows the PK curves of Compound 1 in Formulations 2-2, 6-1, and 11-2, respectively, after subcutaneous administration to SD rats.
FIG. 4 shows the PK curves of comparative compound PF-06882961 in Formulation 4 after subcutaneous administration to SD rats.

### Specific Examples

### Preparation of Compounds and Salts

Compound 1 was prepared according to the process disclosed in CN109790161A. The compound of Formula II was prepared according to the process disclosed in CN115698003A. Compound 6 was prepared according to the process disclosed in CN116390926A.

### Preparation of Sodium Salt:

1.0 g of the compound was taken and dissolved in THF (10 mL). A solution of sodium hydroxide in ethanol (46.2 mg, 1 mL) was added dropwise, and the mixture was stirred at room temperature for 1 hour. The resulting mixture was concentrated to dryness and then crystallized with 10 mL of methyl tert-butyl ether to afford the target compound as a white powder (930 mg).

### Preparation of Calcium Salt:

1.0 g of the compound was added to ethanol (10 mL), and a calcium hydroxide solution (66 mg, 0.5 mL) was added dropwise. The mixture was stirred at room temperature until the solution became clear. Solids precipitated after approximately 1 day. The mixture was filtered to obtain the target calcium salt.

Other salts can be obtained by the steps described above to yield the corresponding target products.

### Example 1

### Preparation of Oil Solution

The active compound (i.e., API) of the present application was dissolved in lipophilic carrier solutions, respectively. The lipophilic carrier comprised castor oil, medium-chain triglycerides (MCT), benzyl benzoate, benzyl alcohol, and ethanol. Oil solution formulations were prepared by an ultrasonic dissolution method, as shown in Table 1.

**Table 1**

| Formulation | | Formulation 1 | Formulation 2-1 | Formulation 2-2 | Formulation 3 | Formulation 4 |
|---|---|---|---|---|---|---|
| API | Main drug | 50mg/mL | 25mg/mL | 25mg/mL | 50mg/mL | 30mg/mL |
| Castor oil | Solvent | 70% | 75% | / | / | / |
| MCT | Solvent | / | / | / | 70% | 80% |
| Soybean oil | Solvent | / | / | 35% | / | / |
| Benzyl benzoate | Solvent | 28% | 15% | 15% | / | / |
| Benzyl alcohol | Solvent | 2% | / | / | 30% | 20% |
| Ethanol | Solvent | / | 10% | 50% | / | / |
| Formulation State | | Suspension | Solution | Solution | Solution | Solution |

### Example 2

### Preparation of Hydrophilic Solution

The active compound (i.e., API) of the present application was dissolved in hydrophilic solvents, respectively. The hydrophilic solvent comprised PEG300, PEG400, ethanol, water, and benzyl alcohol. Hydrophilic solution formulations were prepared by an ultrasonic dissolution method, as shown in Table 2.

**Table 2**

| Formulation | | Formulation 5-1 | Formulation 5-2 | Formulation 6-1 | Formulation 6-2 | Formulation 7 | Formulation 8 | Formulation 9 | Formulation 10 |
|---|---|---|---|---|---|---|---|---|---|
| API | Main drug | 50mg/mL | 50mg/mL | 30mg/mL | 30mg/mL | 50mg/mL | 50mg/mL | 50mg/mL | 75mg/mL |
| PEG300 | Solvent | 100% | 60% | / | 70% | / | 90% | 85% | 90% |
| PEG400 | Solvent | / | / | / | / | 90% | / | / | / |
| Propylene glycol | Solvent | / | / | 50% | / | / | / | / | / |
| Ethanol | Solvent | / | / | / | / | 10% | / | 15% | / |
| Water | Solvent | / | / | / | 30% | / | / | / | / |
| Benzyl alcohol | Solvent | / | 40% | 50% | / | / | 10% | / | 10% |
| Formulation State | | Solution | Solution | Solution | Suspension | Solution | Solution | Solution | Solution |

### Example 3

### Preparation of Micro- or Nano-Suspension

The active compound (i.e., API) of the present application was dispersed in an aqueous solution containing a stabilizer, and was prepared into a micro- or nano-injectable suspension via a media milling process. The stabilizer is selected from the group consisting of polyethylene glycol 3350, Poloxamer 188, Poloxamer 338, and Poloxamer 407. The formulations were prepared according to the table below, in which the following suspensions were obtained by controlling the rotation speed and duration of the media milling, as shown in Table 3.

**Table 3**

| | Formulation | Formulation 11-1 | Formulation 11-2 | Formulation 12 | Formulation 13 |
|---|---|---|---|---|---|
| Formulation components | Name | Amount | Amount | Amount | Amount |
| | API | 30mg | 30mg | 30mg | 50mg |
| | PEG3350 | 20mg | / | 20mg | 20mg |
| | Hydroxypropyl methylcellulose | / | 40mg | / | / |
| | Poloxamer 188 | 40mg | / | / | / |
| | Poloxamer 388 | / | / | 40mg | / |
| | Poloxamer 407 | / | / | / | 60mg |
| | Water for injection | To 1mL | To 1mL | To 1mL | To 1mL |
| Average particle size of the suspension/nm | | 1000 | 1250 | 600 | 300 |

### Example 4

### Preparation of Polymer Gel Solutions

The active compound (i.e., API) of the present application was dispersed in biodegradable polymer solutions, respectively. Polymer gel solution formulations were prepared by an ultrasonic dissolution method, in which the excipients for the polymer gel solutions were selected from the group consisting of poly(lactic-co-glycolic acid) (PLGA), polylactic acid (PLA), and N-methylpyrrolidone (NMP), as shown in Table 4.

**Table 4**

| Formulation | | Formulation 14 | Formulation 15 | Formulation 16 |
|---|---|---|---|---|
| API | Main drug | 50mg/mL | 200mg/mL | 100mg/mL |
| PLGA | Polymers | 300mg/mL | 200mg/mL | / |
| PLA | Polymers | / | / | 100mg/mL |
| NMP | Solvent | 100% | 100% | 100% |
| Formulation State | | Solution | Solution | Solution |

The formulation involved in the present application is a long-acting injection (LAI), which refers to a formulation administered via injection that achieves sustained-release and long-acting effects. Compared to oral formulations, long-acting injectables exhibit stable plasma concentrations, a prolonged effective duration of drug action lasting for several weeks or even months, and a reduced frequency of administration. The purposeful development of such formulations not only provides convenience for patient treatment but yields substantial social impact.

### Example 5

### Different Injections Administered Subcutaneously in SD Rats

Formulation samples comprising Compound 1 of the present application, prepared according to Formulation 4 of Example 1, Formulation 8 of Example 2, and Formulation 12 of Example 3, respectively, were administered to SD rats via subcutaneous (SC) injection, as shown in Table 5 below.

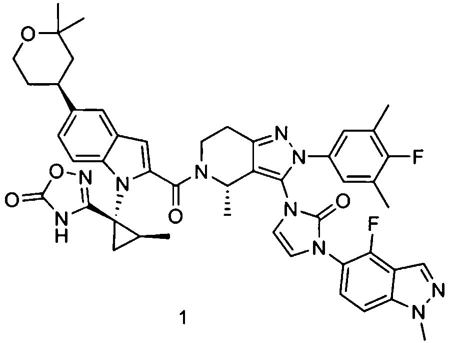

**Table 5**

| No. | Formulation 4 | Formulation 8 | Formulation 12 |
|---|---|---|---|
| Formulation | Oil Solution | Hydrophilic solution | Suspension |
| Concentration | 30mg/mL | 50mg/mL | 30mg/mL |
| Volume of administration | 1mL/kg | 1mL/kg | 1mL/kg |
| Dosage of administration | 30mg/kg | 50mg/kg | 30mg/kg |
| Route of administration | SC | SC | SC |

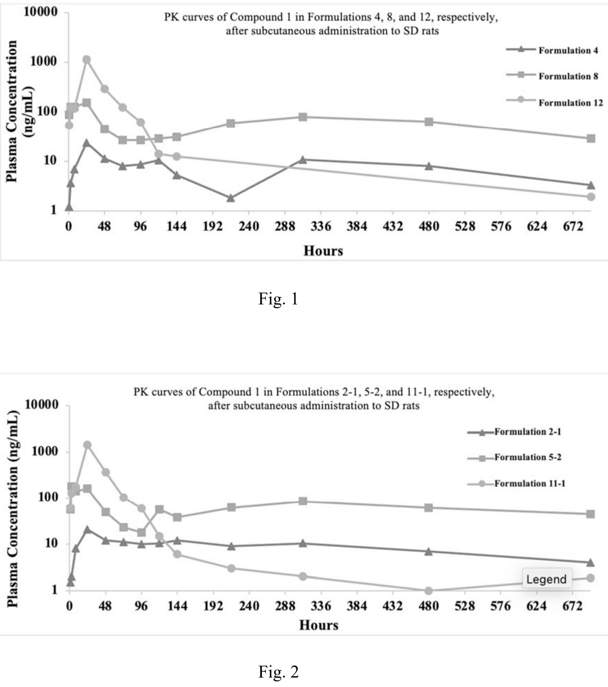

Experimental results were shown in Table 6 and Fig. 1.

| No. | Formulation 4 | Formulation 8 | Formulation 12 |
|---|---|---|---|
| AUC₀₋₆₉₆ₕ (hr*ng/mL) | AUC₀₋₆₉₆ₕ 5390±1110 | AUC₀₋₆₉₆ₕ 39941±5606 | AUC₀₋₆₉₆ₕ 37479±9522 |
| LLOQ | 1ng/ml | 1ng/ml | 1ng/ml |

Each of Formulations 4, 8, and 12 exhibited significant beneficial effects, such as desirable therapeutic effective plasma concentrations, relatively small fluctuations in plasma concentration, relatively stable plasma levels, and a prolonged duration of action, thereby satisfying the requirements for long-acting delivery efficacy.

### Example 6

### Different Injections Administered Subcutaneously in SD Rats

Formulation samples comprising Compound 1 of the present application (i.e. API), prepared according to Formulation 2-1 of Example 1, Formulation 5-2 of Example 2, and Formulation 11-1 of Example 3, respectively, were administered to SD rats via subcutaneous (SC) injection, as shown in Table 7 below.

**Table 7**

| No. | Formulation 2-1 | Formulation 5-2 | Formulation 11-1 |
|---|---|---|---|
| Formulation | Oil Solution | Hydrophilic solution | Suspension |
| Concentration | 25mg/mL | 50mg/mL | 30mg/mL |
| Volume of administration | 1mL/kg | 1mL/kg | 1mL/kg |
| Dosage of administration | 25mg/kg | 50mg/kg | 30mg/kg |
| Route of administration | SC | SC | SC |

Experimental results were shown in Table 8 and Fig. 2.

**Table 8**

| No. | Formulation 2-1 | Formulation 5-2 | Formulation 11-1 |
|---|---|---|---|
| AUC₀₋₆₉₆ₕ (hr*ng/mL) | AUC₀₋₆₉₆ₕ 6038±1711 | AUC₀₋₆₉₆ₕ 43756±7154 | AUC₀₋₆₉₆ₕ 45354±18205 |
| LLOQ | 1ng/ml | 1ng/ml | 1ng/ml |

Each of Formulations 2-1, 5-2, and 11-1 exhibited significant beneficial effects, such as desirable therapeutic effective plasma concentrations, relatively small fluctuations in plasma concentration, relatively stable plasma levels, and a prolonged duration of action, thereby satisfying the requirements for long-acting delivery efficacy.

### Example 7

### Different Injections Administered Subcutaneously in SD Rats

Formulation samples comprising Compound 1 of the present application, prepared according to Formulation 2-2 of Example 1, Formulation 6-1 of Example 2, and Formulation 11-2 of Example 3, respectively, were administered to SD rats via subcutaneous (SC) injection, as shown in Table 9 below.

| No. | Formulation 2-2 | Formulation 6-1 | Formulation 11-2 |
|---|---|---|---|
| Formulation | Oil Solution | Hydrophilic solution | Suspension |
| Concentration | 25mg/mL | 30mg/mL | 30mg/mL |
| Volume of administration | 1mL/kg | 1mL/kg | 1mL/kg |
| Dosage of administration | 25mg/kg | 30mg/kg | 30mg/kg |
| Route of administration | SC | SC | SC |

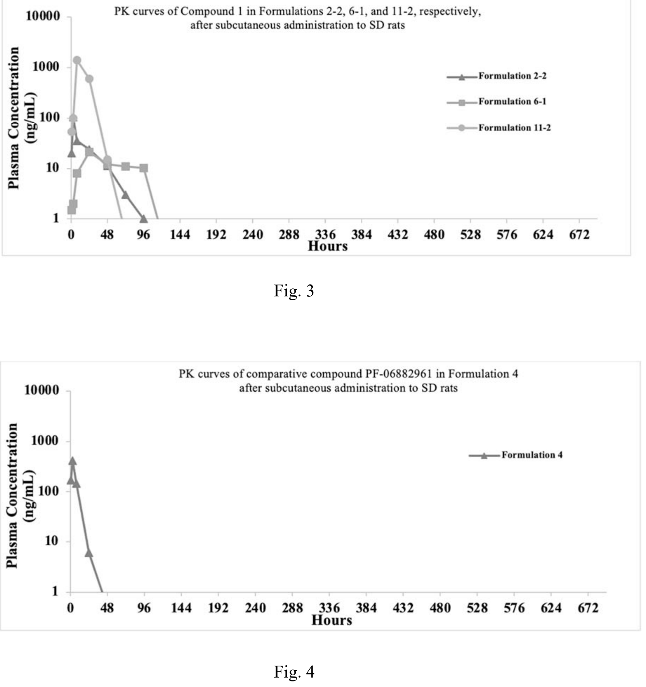

Experimental results were shown in Table 10 and Fig. 3.

**Table 10**

| No. | Formulation 2-2 | Formulation 6-1 | Formulation 11-2 |
|---|---|---|---|
| AUC₀₋₆₉₆ₕ (hr*ng/mL) | AUC₀₋₆₉₆ₕ 1589±620 | AUC₀₋₆₉₆ₕ 1186±327 | AUC₀₋₆₉₆ₕ 27462±1420 |
| LLOQ | 1ng/ml | 1ng/ml | 1ng/ml |

For Formulations 2-2, 6-1, and 11-2, plasma concentrations were not detectable in SD rats (<LLOQ, where LLOQ=1ng/mL) at 96 hours post-administration, failing to achieve a long-acting delivery effect. Meanwhile, the AUC₀₋₆₉₆ₕ was significantly reduced, which was inferior to the *in vivo* results of the corresponding formulations in Example 5 and Example 6.

### Comparative Example 1

### Preparation of Oil Solution of Comparative Compound PF-06882961

The comparative compound PF-06882961 (obtained by the process published in CN 110325530 B) was dissolved in the lipophilic carrier solution. The lipophilic carrier comprised castor oil, medium-chain triglycerides (MCT), benzyl benzoate, benzyl alcohol, medium-chain triglycerides (MCT) in ethanol, and benzyl alcohol. Oil solution formulation was prepared by an ultrasonic dissolution method (referring to Formulation 4).

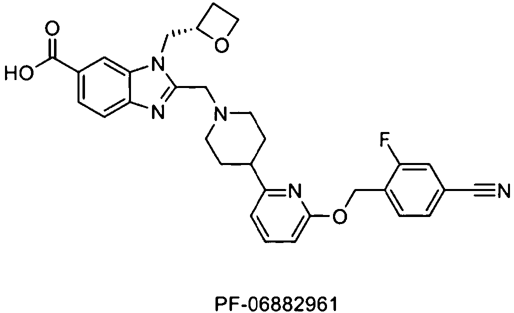

**Table 11**

| Formulation | | Formulation 4 |
|---|---|---|
| API | Main drug | 30mg/mL |
| Castor oil | Solvent | / |
| MCT | Solvent | 80% |
| Benzyl benzoate | Solvent | / |
| Benzyl alcohol | Solvent | 20% |
| Ethanol | Solvent | / |
| Formulation State | | Solution |

### Comparative Example 2

### Injection of Formulation 4 Comprising Comparative Compound PF-06882961 Administered Subcutaneously in SD Rats

The injectable formulation 4 sample of PF-06882961, prepared according to Comparative Example 1, was selected and administered to SD rats via subcutaneous (SC) injection, as shown in Table 12 below.

**Table 12**

| No. | Formulation 4 |
|---|---|
| Formulation | Oil Solution |
| Concentration | 30mg/mL |
| Volume of administration | 1mL/kg |
| Dosage of administration | 30mg/kg |
| Route of administration | SC |

Experimental results were shown in Table 13 and Fig. 4.

**Table 13**

| No. | Formulation 4 |
|---|---|
| AUC₀₋₆₉₆ₕ (hr*ng/mL) | AUC₀₋₆₉₆ₕ 3310±309 |
| LLOQ | 1ng/ml |

At 24 hours post-administration, plasma concentrations were not detectable in the SD rats (<LLOQ, where LLOQ=1ng/mL), failing to achieve a long-acting delivery effect.

## Claims

1. A long-acting injectable formulation, comprising an active compound and at least a pharmaceutically acceptable carrier, wherein the active compound is a compound of Formula (I), stereoisomers, pharmaceutically acceptable salts or deuterated compounds thereof: 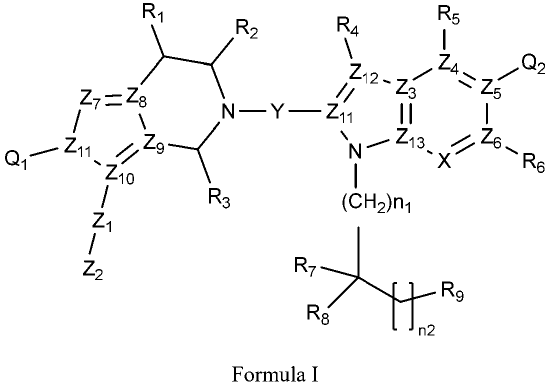 wherein
X is selected from the group consisting of -N= and -CR^{a}, wherein R^{a} is selected from the group consisting of hydrogen atom, halogen and C₁₋₆alkyl;
each of Z₃, Z₄, Z₅, Z₆, Z₇, Z₈, Z₉, Z₁₀, Z₁₁, Z₁₂ and Z₁₃ is independently selected from the group consisting of -N= and -CR^{a};
Q₁ is independently selected from the group consisting of C₆₋₁₀ aryl or 5-to 10-membered heteroaryl; wherein C₆₋₁₀aryl or 5- to 10-membered heteroaryl is optionally substituted by 1 to 5 substituents independently selected from the group consisting of halogen, C₁₋₆alkyl, C₁₋₆haloalkyl, and C₁₋₆alkoxy;
Q₂ is independently selected from the group consisting of 3- to 12-membered heterocyclic group and 5- to 10-membered heteroaryl group, wherein 3-to 12-membered heterocyclic group and 5- to 10-membered are optionally substituted by 1 to 3 substituents independently selected from the group consisting of halogen atoms, C₁₋₆alkyl, C₁₋₆haloalkyl, C₁₋₆alkoxy, and -NR^{Qa}R^{Qb}; and two C₁₋₆ alkyl groups together with carbon atoms to which they are attached form C₃₋₈carbon ring; and wherein R^{Qa} and R^{Qb} are each independently selected from the group consisting of hydrogen, C₁₋₆alkyl, C₁₋₆haloalkyl, and (C₁₋₆alkyl)carbonyl;
each of R₁, R₂ and R₃ is independently selected from the group consisting of hydrogen and C₁₋₆alkyl, wherein C₁₋₆alkyl is optionally substituted by one or more substituents each independently selected from the group consisting of halogen, C₁₋₆alkoxy and -OH;
each of R₄, R₅ and R₆ is independently selected from the group consisting of hydrogen, halogen and C₁₋₆alkyl;
each of R₇ and R₈ is independently selected from the group consisting of hydrogen and C₁₋₆alkyl, wherein C₁₋₆alkyl is optionally substituted by one or more substituents independently selected from the group consisting of halogen and C₃₋₁₅cycloalkyl, or wherein R₇ and R₈ together with carbon atoms to which they are attached form C₃₋₁₅carbocyclic ring; wherein C₃₋₁₅carbocyclic ring formed by R₇ and R₈ together is optionally substituted by 1 to 3 C₁₋₆alkyl, wherein C₁₋₆alkyl is optionally substituted by one or more substituents independently selected from the group consisting of halogen, hydroxyl, -NR^{7a}R^{7b}, C₁₋₆alkoxy, and 3- to 12-membered heterocyclic group, and wherein each of R^{7a} and R^{7b} is independently selected from the group consisting of hydrogen, C₁₋₆alkyl, and (C₁₋₆ alkyl)carbonyl;
n₁ is 0, 1, 2, or 3;
n₂ is 0, 1, 2, 3, 4 or 5;
R₉ is selected from the group consisting of 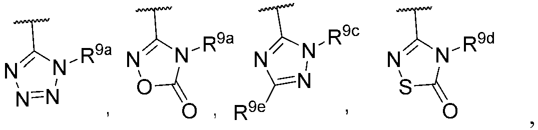 -CO₂R^{9f} and -C(=O)-NR^{9g}R^{9h}; and each of R^{9a}, R^{9b}, R^{9c}, R^{9d}, and R^{9g} is independently selected from the group consisting of hydrogen, C₁₋₆alkyl, and (C₁₋₆alkyl)carbonyl; wherein C₁₋₆alkyl group is optionally substituted by one or more substituents independently selected from the group consisting of halogen and C₁₋₆alkoxy, R^{9e} is hydrogen or C₁₋₆alkyl optionally substituted by one or more halogen atoms; wherein R^{9f} is independently selected from the group consisting of hydrogen and C₁₋₆alkyl; wherein R^{9h} is independently selected from the group consisting of hydrogen, C₁₋₆alkyl, (C₁₋₆alkyl)carbonyl, cyano, and -S(=O)ₙ₃-R⁹ⁱ; wherein n₃ is 0, 1 or 2; and R⁹ⁱ is C₁₋₆alkyl;
Z₁ is selected from the group consisting of 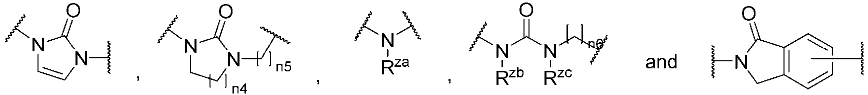
wherein R^{za} is selected from the group consisting of hydrogen, C₁₋₆alkyl and (C₁₋₆alkyl)carbonyl, each of R^{zb} and R^{zc} is independently selected from the group consisting of hydrogen and C₁₋₆alkyl; n₄ is 1, 2 or 3; and n₅ and n₆ are independently integers from 0 to 10;
Z₂ is selected from the group consisting of C₁₋₆alkyl, C₃₋₁₅cycloalkyl, 3-to 12-membered heterocyclyl, C₆₋₁₀aryl, and 5- to 10-membered heteroaryl, wherein C₃₋₁₅cycloalkyl, 3- to 12-membered heterocyclyl, C₆₋₁₀aryl, and 5- to 10-membered heteroaryl are independently and optionally substituted by one to five G; wherein G is selected from the group consisting of:
a. oxo;
b. halogen;
c. cyano;
d. -NR^{zd}R^{ze}; wherein R^{zd} and R^{ze} are independently selected from the group consisting of hydrogen, C₁₋₆alkyl, and (C₁₋₆alkyl)carbonyl; wherein C₁₋₆ alkyl is optionally substituted by one or more substituents independently selected from the group consisting of hydroxyl, halogen, and C₁₋₆alkoxy;
e. -C(=O)-NR^{zf}R^{zg}; wherein each of R^{zf} and R^{zg} is independently selected from the group consisting of hydrogen, C₁₋₆alkyl, and (C₁₋₆alkyl)carbonyl; wherein C₁₋₆alkyl is optionally substituted by one or more substituents independently selected from the group consisting of hydroxyl, halogen, and C₁₋₆alkoxy;
f. -S(=O)ₙ₇-R^{zh}; wherein n₇ is 0, 1 or 2; R^{zh} is independently selected from the group consisting of hydrogen and C₁₋₆alkyl;
g. C₁₋₆alkyl; wherein the C₁₋₆alkyl is optionally substituted by one or more substituents independently selected from the group consisting of halogen, hydroxyl, -NR^{zi}R^{zj}, C₁₋₆alkoxy, and 3- to 12-membered heterocyclyl; wherein each of R^{zi} and R^{zj} is independently selected from the group consisting of hydrogen or C₁₋₆alkyl, and wherein 3- to 12-membered heterocyclyl is optionally substituted by one or more cyano, C₁₋₆alkyl and 3- to 12-membered heterocyclyl;
h. C₁₋₆alkoxy; wherein C₁₋₆alkoxy is optionally substituted by one or more hydroxyl, halogen and C₁₋₆alkoxy;
i. 3- to 12-membered heterocyclic; wherein 3- to 12-membered heterocyclic is optionally substituted by one or more substituents independently selected from the group consisting of C₁₋₆alkyl and (C₁₋₆alkyl)carbonyl;
j. C₆₋₁₀aryl; wherein C₆₋₁₀aryl is optionally substituted by one or more (C₁₋₆ alkyl)carbonyl ; and
k. 5- to 10-membered heteroaryl; wherein 5- to 10-membered heteroaryl is optionally substituted by one or more substituents independently selected from C₁₋₆alkyl, C₁₋₆alkoxy, -NR^{zk}R^{zl}, and 3- to 12-membered heterocyclic; wherein R^{zk} and R^{zl} are independently selected from the group consisting of hydrogen, C₁₋₆alkyl, and (C₁₋₆alkyl)carbonyl, and wherein 3- to 12-membered heterocyclic is optionally substituted by one or more substituents independently selected from the group consisting of C₁₋₆alkyl and C₁₋₆alkoxy.

2. A long-acting injectable formulation, comprising an active compound and at least a pharmaceutically acceptable carrier, wherein the active compound is a compound of Formula (II), stereoisomers, pharmaceutically acceptable salts or deuterated compounds thereof: 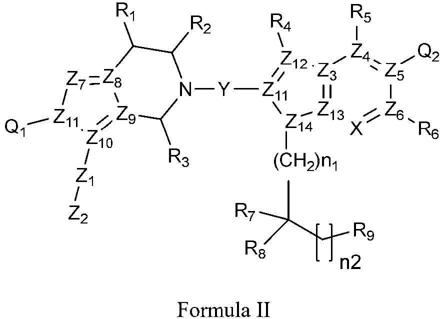 wherein
X is selected from the group consisting of -N= and -CR^{a}, wherein R^{a} is selected from the group consisting of hydrogen atom, halogen and C₁₋₆alkyl;
each of Z₃, Z₄, Z₅, Z₆, Z₇, Z₈, Z₉, Z₁₀, Z₁₁, Z₁₂ and Z₁₃ is independently selected from the group consisting of -N= and -CR^{a};
Q₁ is independently selected from the group consisting of C₆₋₁₀ aryl or 5-to 10-membered heteroaryl; wherein C₆₋₁₀aryl or 5- to 10-membered heteroaryl is optionally substituted by 1 to 5 substituents independently selected from the group consisting of halogen, C₁₋₆alkyl, C₁₋₆haloalkyl, and C₁₋₆alkoxy;
Q₂ is independently selected from the group consisting of 3- to 12-membered heterocyclic group and 5- to 10-membered heteroaryl group, wherein 3-to 12-membered heterocyclic group and 5- to 10-membered are optionally substituted by 1 to 3 substituents independently selected from the group consisting of halogen atoms, C₁₋₆alkyl, C₁₋₆haloalkyl, C₁₋₆alkoxy, and -NR^{Qa}R^{Qb}; and two C₁₋₆ alkyl groups together with carbon atoms to which they are attached form C₃₋₈carbon ring; and wherein R^{Qa} and R^{Qb} are each independently selected from the group consisting of hydrogen, C₁₋₆alkyl, C₁₋₆haloalkyl, and (C₁₋₆alkyl)carbonyl;
each of R₁, R₂ and R₃ is independently selected from the group consisting of hydrogen and C₁₋₆alkyl, wherein C₁₋₆alkyl is optionally substituted by one or more substituents each independently selected from the group consisting of halogen, C₁₋₆alkoxy and -OH;
each of R₄, R₅ and R₆ is independently selected from the group consisting of hydrogen, halogen and C₁₋₆alkyl;
each of R₇ and R₈ is independently selected from the group consisting of hydrogen and C₁₋₆alkyl, wherein C₁₋₆alkyl is optionally substituted by one or more substituents independently selected from the group consisting of halogen and C₃₋₁₅cycloalkyl, or wherein R₇ and R₈ together with carbon atoms to which they are attached form C₃₋₁₅carbocyclic ring; wherein C₃₋₁₅carbocyclic ring formed by R₇ and R₈ together is optionally substituted by 1 to 3 C₁₋₆alkyl, wherein C₁₋₆alkyl is optionally substituted by one or more substituents independently selected from the group consisting of halogen, hydroxyl, -NR^{7a}R^{7b}, C₁₋₆alkoxy, and 3- to 12-membered heterocyclic group, and wherein each of R^{7a} and R^{7b} is independently selected from the group consisting of hydrogen, C₁₋₆alkyl, and (C₁₋₆ alkyl)carbonyl;
n₁ is 0, 1, 2, or 3;
n₂ is 0, 1, 2, 3, 4 or 5;
R₉ is selected from the group consisting of 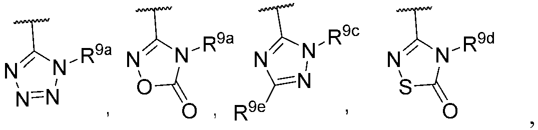 -CO₂R^{9f} and -C(=O)-NR^{9g}R^{9h}; and each of R^{9a}, R^{9b}, R^{9c}, R^{9d}, and R^{9g} is independently selected from the group consisting of hydrogen, C₁₋₆alkyl, and (C₁₋₆alkyl)carbonyl; wherein C₁₋₆alkyl group is optionally substituted by one or more substituents independently selected from the group consisting of halogen and C₁₋₆alkoxy, R^{9e} is hydrogen or C₁₋₆alkyl optionally substituted by one or more halogen atoms; wherein R^{9f} is independently selected from the group consisting of hydrogen and C₁₋₆alkyl; wherein R^{9h} is independently selected from the group consisting of hydrogen, C₁₋₆alkyl, (C₁₋₆alkyl)carbonyl, cyano, and -S(=O)ₙ₃-R⁹ⁱ; wherein n₃ is 0, 1 or 2; and R⁹ⁱ is C₁₋₆alkyl;
Z₁ is selected from the group consisting of 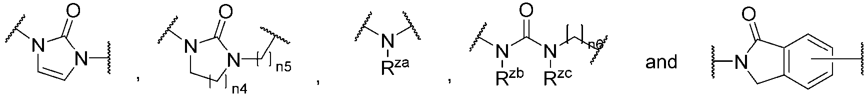
wherein R^{za} is selected from the group consisting of hydrogen, C₁₋₆alkyl and (C₁₋₆alkyl)carbonyl, each of R^{zb} and R^{zc} is independently selected from the group consisting of hydrogen and C₁₋₆alkyl; n₄ is 1, 2 or 3; and n₅ and n₆ are independently integers from 0 to 10;
Z₂ is selected from the group consisting of C₁₋₆alkyl, C₃₋₁₅cycloalkyl, 3-to 12-membered heterocyclyl, C₆₋₁₀aryl, and 5- to 10-membered heteroaryl, wherein C₃₋₁₅cycloalkyl, 3- to 12-membered heterocyclyl, C₆₋₁₀aryl, and 5- to 10-membered heteroaryl are independently and optionally substituted by one to five G; wherein G is selected from the group consisting of:
a. oxo;
b. halogen;
c. cyano;
d. -NR^{zd}R^{ze}; wherein R^{zd} and R^{ze} are independently selected from the group consisting of hydrogen, C₁₋₆alkyl, and (C₁₋₆alkyl)carbonyl; wherein C₁₋₆ alkyl is optionally substituted by one or more substituents independently selected from the group consisting of hydroxyl, halogen, and C₁₋₆alkoxy;
e. -C(=O)-NR^{zf}R^{zg}; wherein each of R^{zf} and R^{zg} is independently selected from the group consisting of hydrogen, C₁₋₆alkyl, and (C₁₋₆alkyl)carbonyl; wherein C₁₋₆alkyl is optionally substituted by one or more substituents independently selected from the group consisting of hydroxyl, halogen, and C₁₋₆alkoxy;
f. -S(=O)ₙ₇-R^{zh}; wherein n₇ is 0, 1 or 2; R^{zh} is independently selected from the group consisting of hydrogen and C₁₋₆alkyl;
g. C₁₋₆alkyl; wherein the C₁₋₆alkyl is optionally substituted by one or more substituents independently selected from the group consisting of halogen, hydroxyl, -NR^{zi}R^{zj}, C₁₋₆alkoxy, and 3- to 12-membered heterocyclyl; wherein each of R^{zi} and R^{zj} is independently selected from the group consisting of hydrogen or C₁₋₆alkyl, and wherein 3- to 12-membered heterocyclyl is optionally substituted by one or more cyano, C₁₋₆alkyl and 3- to 12-membered heterocyclyl;
h. C₁₋₆alkoxy; wherein C₁₋₆alkoxy is optionally substituted by one or more hydroxyl, halogen and C₁₋₆alkoxy;
i. 3- to 12-membered heterocyclic; wherein 3- to 12-membered heterocyclic is optionally substituted by one or more substituents independently selected from the group consisting of C₁₋₆alkyl and (C₁₋₆alkyl)carbonyl;
j. C₆₋₁₀aryl; wherein C₆₋₁₀aryl is optionally substituted by one or more (C₁₋₆ alkyl)carbonyl ;
k. 5- to 10-membered heteroaryl; wherein 5- to 10-membered heteroaryl is optionally substituted by one or more substituents independently selected from C₁₋₆alkyl, C₁₋₆alkoxy, -NR^{zk}R^{zl}, and 3- to 12-membered heterocyclic; wherein R^{zk} and R^{z1} are independently selected from the group consisting of hydrogen, C₁₋₆alkyl, and (C₁₋₆alkyl)carbonyl, and wherein 3- to 12-membered heterocyclic is optionally substituted by one or more substituents independently selected from the group consisting of C₁₋₆alkyl and C₁₋₆alkoxy; and
l. -P(O)R_{g1}R_{g2}; wherein R_{g1} and R_{g2} are each independently selected from the group consisting of hydrogen, C₁₋₆alkyl, and C₃₋₈cycloalkyl.

3. The formulation of claim 1, wherein the compound of Formula (I) has a structure of formula (III):

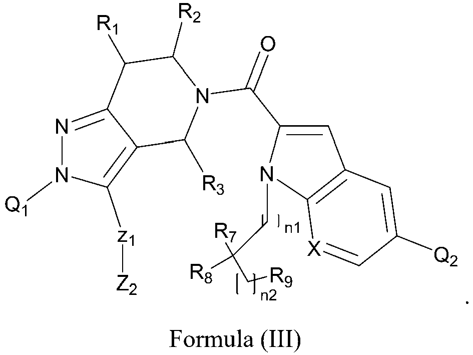

4. The formulation of claim 3, wherein the compound of Formula (III) has a structure of formula (V): 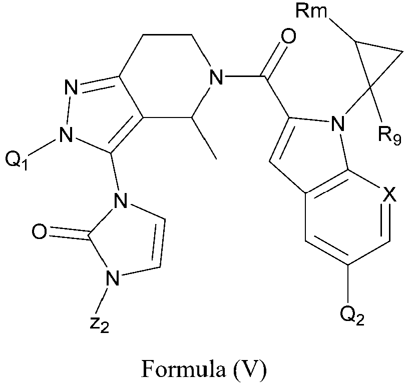 wherein Rm is C₁₋₆alkyl.

5. The formulation of claim 2, wherein the compound of Formula (II) has a structure of formula (IV):

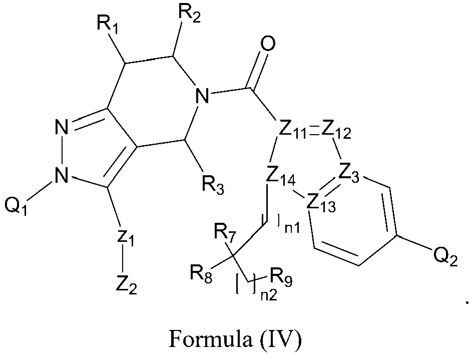

6. The formulation of claim 5, wherein the compound of Formula (IV) has a structure of formula (VI):

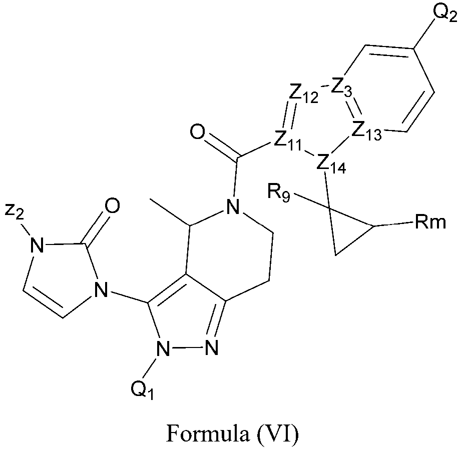

7. The formulation of claim 2, wherein the compound of Formula (II) is selected from the group consisting of: 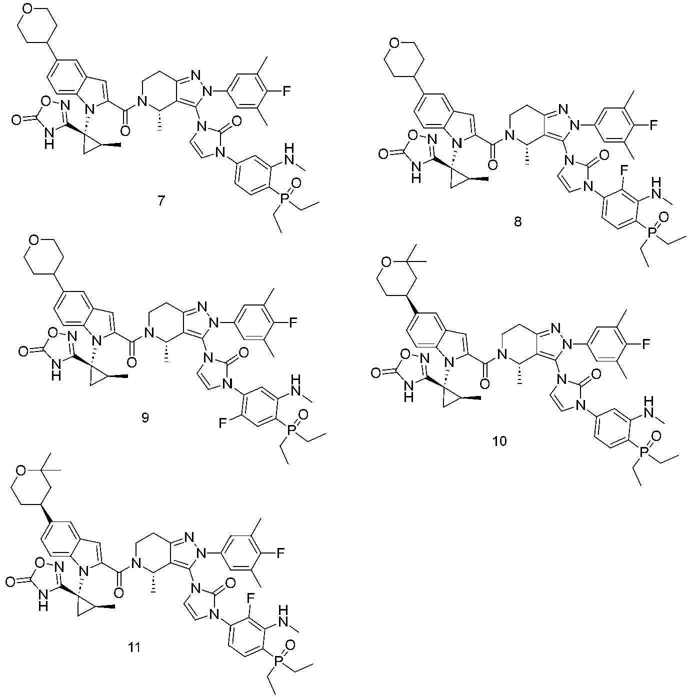 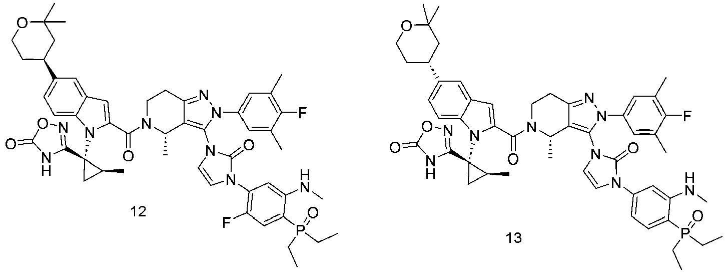 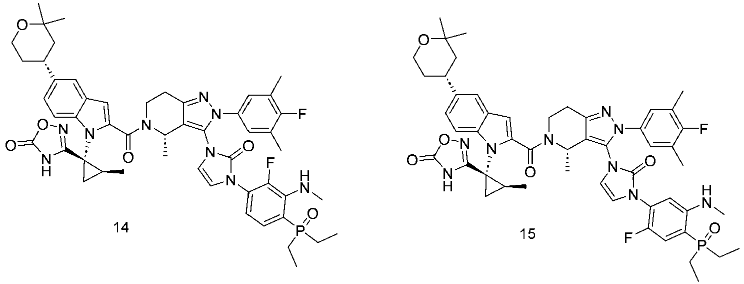 or stereoisomers, pharmaceutically acceptable salts or deuterated compounds thereof and at least one pharmaceutically acceptable carrier; preferably the pharmaceutically acceptable salts are selected from the group consisting of sodium salts, potassium salts, calcium salts, and magnesium salts.

8. The formulation of claim 1, wherein the active compound is compound 1: 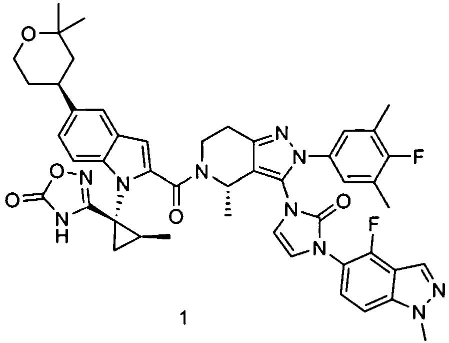 or stereoisomers, pharmaceutically acceptable salts or deuterated compounds thereof and at least one pharmaceutically acceptable carrier; preferably the pharmaceutically acceptable salts are selected from the group consisting of:
(1) sodium salts, comprising compound 2: 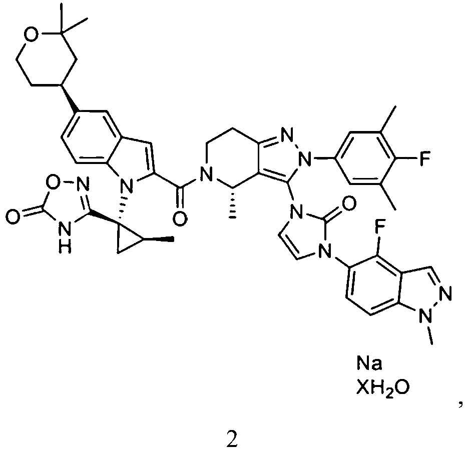 wherein X is number of crystal water and selected from the group consisting of 0, 0.5, 1, 2, and 3;
(2) potassium salts, comprising compound 3: 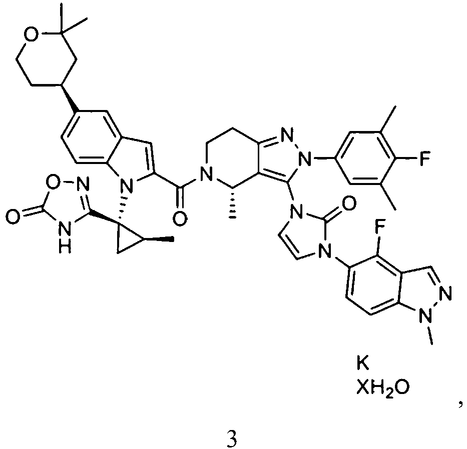 wherein X is number of crystal water and selected from the group consisting of 0, 0.5, 1, 2, and 3;
(3) calcium salts, comprising compound 4: 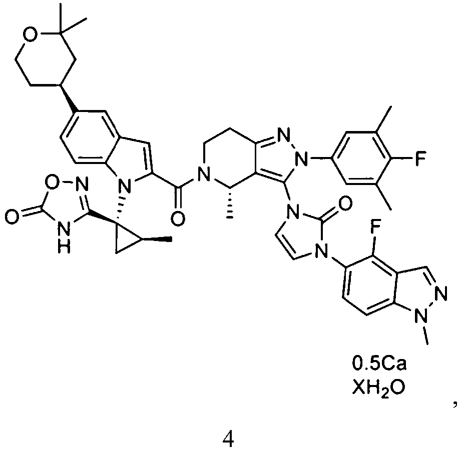 wherein X is number of crystal water and selected from the group consisting of 0, 0.5, 1, 2, and 3; and
(4) magnesium salts, comprising compound 5: 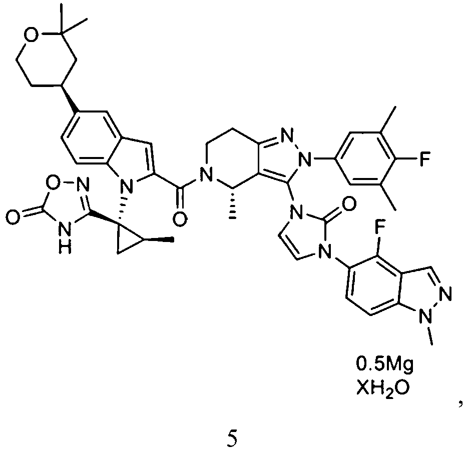 wherein X is number of crystal water and selected from the group consisting of 0, 0.5, 1, 2, and 3.

9. A long-acting injectable formulation, comprising an active compound and at least a pharmaceutically acceptable carrier, wherein the active compound is compound 6, stereoisomers, pharmaceutically acceptable salts or deuterated compounds thereof and at least a pharmaceutically acceptable carrier: 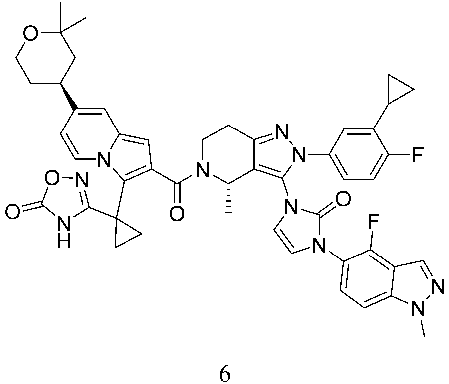 wherein preferably the pharmaceutically acceptable salts are selected from the group consisting of sodium salts, potassium salts, calcium salts, and magnesium salts.

10. The formulation of any one of claims 1 to 9, wherein the active compound is dissolved in a lipophilic carrier solution to form an oil solution, the lipophilic carrier solution comprises an oil solvent and an organic solvent;
preferably the active compound is selected from the group consisting of compounds 1, 6, 7-15, stereoisomers, pharmaceutically acceptable salts, and deuterated compounds thereof, more preferably is compound 1 or pharmaceutically acceptable salts thereof, and most preferably is compounds 1-5;
preferably the oil solvent is selected from the group consisting of castor oil, sesame oil, fatty acid glyceride, benzyl benzoate, benzyl alcohol, and any mixture thereof; more preferably the oil solvent is medium-chain fatty acid glyceride; and
preferably the organic solvent is benzyl alcohol or ethanol; more preferably the organic solvent is benzyl alcohol.

11. The formulation of claim 10, wherein the concentration of the active compound or the pharmaceutically acceptable salts thereof in the formulation is 0.1 to 400 mg/mL, preferably 1 to 200 mg/mL;
wherein weight ratio of the oil solvent to the organic solvent is from 6:4 to 9:1;
preferably weight ratio of the oil solvent to the organic solvent isfrom 7:3 to 9:1; and
more preferably weight ratio of the oil solvent to the organic solvent is 8:2.

12. The formulation of any one of claims 1 to 9, wherein the active compound is dissolved in a hydrophilic solvent to form a hydrophilic solution;
preferably the active compound is selected from the group consisting of compounds 1, 6, 7-15, stereoisomers, pharmaceutically acceptable salts, and deuterated compounds thereof, more preferably is compound 1 or pharmaceutically acceptable salts thereof, most preferably is compounds 1-5;
preferably the hydrophilic solvent comprises one of polyethylene glycol, organic solvent and water or any mixture thereof;
preferably the polyethylene glycol is selected from the group consisting of polyethylene glycol 300 and polyethylene glycol 400; more preferably the polyethylene glycol is polyethylene glycol 300; and
preferably the organic solvent is selected from the group consisting of benzyl alcohol, ethanol and N-methylpyrrolidone (NMP); more preferably the organic solvent is selected from the group consisting of benzyl alcohol and ethanol.

13. The formulation of claim 12, wherein concentration of the active compound or pharmaceutically acceptable salts thereof in the formulation is 0.1 to 400 mg/mL, preferably 1 to 200 mg/mL;
wherein weight ratio of polyethylene glycol to the organic solvent is from 6:4 to 10:0;
more preferably weight ratio of polyethylene glycol to the organic solvent is from 7:3 to 10:0; even more preferably weight ratio of polyethylene glycol to the organic solvent is 9:1 to 10:0;
preferably weight ratio of polyethylene glycol 300 to the organic solvent is from 6:4 to 10:0; more preferably weight ratio of polyethylene glycol 300 to the organic solvent is from 7:3 to 10:0; even more preferably weight ratio of polyethylene glycol 300 to the organic solvent is 9:1 to 10:0; and
preferably weight ratio of polyethylene glycol 400 to the organic solvent is from 6:4 to 10:0; more preferably weight ratio of polyethylene glycol 400 to the organic solvent is from 7:3 to 10:0; even more preferably weight ratio of polyethylene glycol 400 to the organic solvent is 9:1 to 10:0.

14. The formulation of claim 12, further comprising a pH adjusting agent, wherein the pH adjusting agent is selected from the group consisting of sodium hydroxide, hydrochloric acid, citric acid, sodium citrate, phosphoric acid, sodium hydrogen phosphate, and any mixture thereof; preferably pH of the formulation is 4 to 10.

15. The formulation of claim 12, further comprising a release retardant, wherein the release retardant is selected from the group consisting of poly(lactic-co-glycolic acid) (PLGA) and polylactic acid (PLA), and weight ratio thereof in the hydrophilic solvent is 1% to 10%.

16. The formulation of claims 1-9, wherein the active compound is dispersed in a stabilizer solution to form a suspension of drug-loaded microparticles or nanoparticles;
preferably the active compound is selected from the group consisting of compounds 1, 6, 7-15, stereoisomers, pharmaceutically acceptable salts, and deuterated compounds thereof, more preferably is compound 1 or pharmaceutically acceptable salts thereof, most preferably is compounds 1-5;
wherein the stabilizer solution is selected from the group consisting of one of polyethylene glycol, poloxamer, Tween, vitamin E polyethylene glycol succinate (TPGS), distearoylphosphatidylethanolamine (DSPE)-polyethylene glycol (PEG), polyvinylpyrrolidone, mannitol, hydroxypropyl methylcellulose, sodium carboxymethylcellulose, methylcellulose; and any combination thereof; preferably the stabilizer is selected from the group consisting of poloxamer and polyethylene glycol, wherein polyethylene glycol is polyethylene glycol 400 to 6000, more preferably polyethylene glycol 1000 to 4000, most preferably polyethylene glycol 3350, and poloxamer is poloxamer 407, poloxamer 338, or poloxamer 188.

17. The formulation of claim 16, wherein concentration of the active compound or pharmaceutically acceptable salts thereof in the formulation is 0.1 to 400 mg/mL, preferably 1 to 200 mg/mL;
wherein weight ratio of poloxamer in the suspension of drug-loaded microparticles or nanoparticles is 1% to 10%,
wherein weight ratio of polyethylene glycol in the suspension of drug-loaded microparticles or nanoparticles is 0.5% to 5%;
preferably active ingredient in the microparticle or nanoparticle suspension exists in form of particles with a median particle size of about 0.1 to 10 µm; more preferably the median particle size range of the suspension of drug-loaded microparticles is 1 to 10 µm; even more preferably the median particle size range of the suspension of drug-loaded nanoparticles is 100 to 1000 nm.

18. The formulation of any one of claims 1-9, wherein the active compound is dispersed in a biodegradable polymer solution to prepare a polymer gel solution formulation, the polymer gel solution comprising a polymer gel material and an organic solvent;
preferably the active compound is selected from the group consisting of compounds 1, 6, 7-15, stereoisomers, pharmaceutically acceptable salts, and deuterated compounds thereof, more preferably is compound 1 or pharmaceutically acceptable salts thereof, most preferably is compounds 1-5;
wherein preferably the polymer gel material is selected from the group consisting of poly(lactic-co-glycolic acid) (PLGA), polylactic acid (PLA), polyoxyethylene-polycaprolactone block copolymer, and polyoxyethylene-polyoxypropylene block copolymer;
preferably the polymer gel material is poly(lactic-co-glycolic acid) (PLGA);
preferably ratio of lactic acid to glycolic acid in the poly(lactic-co-glycolic acid) molecule is 75:25 or 50:50;
preferably intrinsic viscosity of poly(lactic-co-glycolic acid) is 0.1 to 0.3 dL/g;
preferably the organic solvent is selected from the group consisting of N-methylpyrrolidone (NMP), dimethyl sulfoxide (DMSO), benzyl alcohol, benzyl benzoate, and any combination thereof;
preferably concentration of the active compound or pharmaceutically acceptable salts thereof in the formulation is 0.1 to 400 mg/mL, preferably 1 to 200 mg/mL;
preferably weight ratio of the poly(lactic-co-glycolic acid) in the polymer gel solution is 10% to 80%; and
preferably weight ratio of the organic solvent in the polymer gel solution is 20% to 90%.

19. The formulation of any one of claims 1-18, wherein the formulation is suitable for intramuscular or subcutaneous administration.

20. A method for treating GLP-1-mediated diseases or conditions, comprising administering to a subject in need thereof an effective amount of the formulation of any one of claims 1 to 18.

21. The method of claim 11, wherein the GLP-1-mediated diseases or conditions is selected from the group consisting of T1 D, T2DM, prediabetes, idiopathic T1 D, LADA, EOD, YOAD, MODY, malnutrition-associated diabetes mellitus, gestational diabetes mellitus, hyperglycemia, insulin resistance, hepatic insulin resistance, impaired glucose tolerance, diabetic neuropathy, diabetic nephropathy, nephropathy, diabetic retinopathy, adipocyte dysfunction, visceral fat deposition, sleep apnea, obesity, eating disorders, weight gain due to use of other medications, excessive glucose consumption, dyslipidemia, hyperinsulinemia, NAFLD, NASH, fibrosis, cirrhosis, hepatocellular carcinoma, cardiovascular disease, atherosclerosis, coronary artery disease, peripheral vascular disease, hypertension, endothelial dysfunction, impaired vascular compliance, congestive heart failure, myocardial infarction, stroke, hemorrhagic stroke, ischemic stroke, traumatic brain injury, pulmonary hypertension, post angioplasty restenosis, intermittent claudication, postprandial lipemia, metabolic acidosis, ketoacidosis, arthritis, osteoporosis, Parkinson's disease, left ventricular hypertrophy, peripheral arterial disease, macular degeneration, cataract, glomerulosclerosis, chronic renal failure, metabolic syndrome, syndrome X, premenstrual syndrome, angina pectoris, thrombosis, atherosclerosis, transient ischemic attack, revascularization, impaired glucose metabolism, impaired fasting glucose, hyperuricemia, gout, erectile dysfunction, skin and connective tissue disorders, psoriasis, foot ulcers, ulcerative colitis, high apolipoprotein B lipoproteinemia, Alzheimer's disease, schizophrenia, cognitive impairment, inflammatory bowel disease, short bowel syndrome, Crohn's disease, colitis, irritable bowel syndrome, polycystic ovary syndrome and substance addiction.
